# FASCICULE DE BREVET EUROPEEN

(11) **EP 3 119 382 B1**
(45) Date de publication et mention de la délivrance du brevet: **25.03.2020**
(21) Numéro de dépôt: 15715346.1
(22) Date de dépôt: 18.03.2015
(51) Int. Cl.: A61K 8/368, A61K 8/49, A61K 31/05, A61Q 19/08

(54) **APPLICATIONS COSMETIQUES ET PHARMACEUTIQUES DE LA VESCALAGINE ET DE LA CASTALAGINE**
COSMETISCHE UND PHARMAZEUTISCHE ANWENDUNGEN VON VESCALAGINE UND CASTALAGINE
COSMETIC AND PHARMACEUTIC APPLICATIONS OF VESCALAGINE AND CASTALAGINE

(30) Priorité: 18.03.2014 FR 1452205; 14.01.2015 FR 1550295
(43) Date de publication de la demande: 25.01.2017
(73) Titulaire: Greenpharma, 45100 Orléans (FR); Bioalternatives, 86160 Gencay (FR)
(72) Inventeur: BERNARD, Philippe, F-45240 La Ferté Saint Aubin (FR); BERNARD, François-Xavier, F-86160 Saint Maurice La Clouere (FR); HIMBERT, Franck, F-45000 Orléans (FR)
(74) Mandataire: Agasse, Stéphane
(86) Numéro de dépôt international: PCT/FR2015/050664
(87) Numéro de publication internationale: WO 2015/140470

(56) Documents cités:
- WO-A1-2012/144080
- WO-A1-2013/140009
- WO-A1-2014/044591
- DE-A1- 10 140 539
- FR-A1- 2 976 490
- KR-A- 20090 103 823
- KR-A- 20100 055 734
- US-A1- 2010 129 418
- US-A1- 2011 039 796
- A. SINGH ET AL: "Dietary polyphenols in the prevention and treatment of allergic diseases", CLINICAL & EXPERIMENTAL ALLERGY, vol. 41, no. 10, 30 mai 2011 (2011-05-30), pages 1346-1359, XP055049818, ISSN: 0954-7894, DOI: 10.1111/j.1365-2222.2011.03773.x
- AGYARE CHRISTIAN ET AL: "Ellagitannins from Phyllanthus muellerianus (Kuntze) Exell.: Geraniin and furosin stimulate cellular activity, differentiation and collagen synthesis of human skin keratinocytes and dermal fibroblasts.", PHYTOMEDICINE : INTERNATIONAL JOURNAL OF PHYTOTHERAPY AND PHYTOPHARMACOLOGY 15 MAY 2011, vol. 18, no. 7, 15 mai 2011 (2011-05-15), pages 617-624, XP002741452, ISSN: 1618-095X
- DATABASE WPI Week 201312 Thomson Scientific, London, GB; AN 2012-L53513 XP002741453, & KR 2012 0091877 A (ALTORI CO LTD) 20 août 2012 (2012-08-20)
- DATABASE WPI Week 201312 Thomson Scientific, London, GB; AN 2012-Q91904 XP002741454, & KR 2012 0129168 A (UNIV HALLYM IND ACADEMIC COOP FOUND) 28 novembre 2012 (2012-11-28)
- DE PASCALE M CLARA ET AL: "Increased expression of transglutaminase-1 and PPARgamma after vitamin E treatment in human keratinocytes.", ARCHIVES OF BIOCHEMISTRY AND BIOPHYSICS 15 MAR 2006, vol. 447, no. 2, 15 mars 2006 (2006-03-15) , pages 97-106, XP002741455, ISSN: 0003-9861
- HIGUCHI K ET AL: "Sphingosylphosphorylcholine is an activator of transglutaminase activity in human keratinocytes.", JOURNAL OF LIPID RESEARCH OCT 2001, vol. 42, no. 10, octobre 2001 (2001-10), pages 1562-1570, XP002741456, ISSN: 0022-2275
- LIEW F-M ET AL: "Regulation of transglutaminase 1 gene expression by 12-O-tetradecanoylphorbol-13-acetate, dexamethasone, and retinoic acid in cultured human keratinocytes", EXPERIMENTAL CELL RESEARCH, ACADEMIC PRESS, US, vol. 202, no. 2, 1 octobre 1992 (1992-10-01), pages 310-315, XP024791794, ISSN: 0014-4827, DOI: 10.1016/0014-4827(92)90080-R [extrait le 1992-10-01]
- ROSILLO M A ET AL: "Protective effect of ellagic acid, a natural polyphenolic compound, in a murine model of Crohn's disease", BIOCHEMICAL PHARMACOLOGY, ELSEVIER, US, vol. 82, no. 7, 30 juin 2011 (2011-06-30), pages 737-745, XP028263248, ISSN: 0006-2952, DOI: 10.1016/J.BCP.2011.06.043 [extrait le 2011-07-07]
- Y BRUN ET AL: "Experimental study of antidiarrheal activity of Salicairine", FUNDAMENTAL & CLINICAL PHARMACOLOGY, vol. 12, no. 1, 2 janvier 1998 (1998-01-02), pages 30-36, XP55138031, ISSN: 0767-3981, DOI: 10.1111/j.1472-8206.1998.tb00920.x
- Suhad S Humadi ET AL: "LYTHRUM SALICARIA (PURPLE LOOSESTRIFE). MEDICINAL USE, EXTRACTION AND IDENTIFICATION OF ITS TOTAL PHENOLIC COMPOUNDS", FARMACIA, 1 janvier 2009 (2009-01-01), page 192, XP55138035, Extrait de l'Internet: URL:http://www.revistafarmacia.ro/20092/is sue22009art06.pdf [extrait le 2014-09-03]

## Description

La présente invention concerne un composé, ses sels et ses dérivés, ledit composé étant choisi parmi l'acide gallique, l'acide hexahydroxydiphénique, l'acide ellagique et les dérivés de ces acides et notamment les gallotannins et les ellagitannins, ainsi que ses applications cosmétiques et pharmaceutiques.

Les auteurs de l'invention ont en effet découvert que les composés de l'invention possèdent une efficacité pour restaurer la fonction barrière des organes humains ou animaux, qu'ils soient externes comme l'épiderme ou internes comme ceux du tube digestif, permettant de renforcer la protection des organes vis-à-vis des agressions qu'ils subissent, par exemple des agressions environnementales concernant l'épiderme ou des lésions associées à un état pathologique. De telles propriétés sont particulièrement intéressantes pour prévenir ou soigner des affections de la peau telles que l'acné, la dermatite atopique, le psoriasis, l'eczéma, la sécheresse de la peau à tendance atopique, les rougeurs, mais aussi pour préserver les peaux jeunes contre le vieillissement naturel ou prématuré ainsi que les peaux âgées, ainsi que pour prévenir ou traiter des pathologies d'un organe interne ou des conséquences des ces pathologies, comme la maladie de Crohn.

Les auteurs de l'invention ont mis en évidence qu'un composé ci-dessus permet de stimuler l'expression de différents marqueurs protéiques essentiels dans la formation du *stratum corneum* ou couche cornée, dont l'enzyme-clé, la transglutaminase TGK.

Le *stratum corneum* est la partie la plus superficielle de la peau et représente l'aboutissement du processus de différenciation des kératinocytes. Au cours de cette différenciation, les kératinocytes issus de l'assise basale subissent une série de remaniements métaboliques et structuraux tout au long de leur migration vers la surface de la peau. Le dernier stade est leur transformation en cornéocytes dont l'accumulation en une structure cohésive constitue le *stratum corneum* qui assure la fonction de barrière. Tout au long du processus de différenciation, plusieurs protéines interviennent.

L'une d'elles, la TGK, de la famille des glutaminases catalyse l'établissement de liaisons peptidiques covalentes du type ε(γ-glutamyl)-lysine entre divers précurseurs protéiques qui forment le *stratum corneum* et lui confère cette capacité à protéger la peau vis-à-vis d'agressions extérieures et à limiter la diffusion de l'eau provenant de l'intérieur. La transglutaminase forme des polymères de protéines généralement insolubles. Ces polymères biologiques sont indispensables à l'organisme pour créer des barrières et structures stables.

La présente invention vise précisément à offrir de nouvelles compositions capables de stimuler les facteurs intervenant dans la différenciation, appelés facteurs pro-différenciants comme la TGK, permettant ainsi d'améliorer les conditions de la peau, des phanères, ainsi que des muqueuses, sains ou lésés, et donc de renforcer leurs fonctions, mais aussi celles de la paroi d'organes internes.

La maladie de Crohn est une maladie inflammatoire chronique qui peut atteindre l'ensemble du tube digestif. Elle se développe le plus souvent dans les intestins entraînant une altération de la paroi par ulcérations et fissures de la paroi entraînant la perte de sa fonction barrière.

Les dermatoses sont des affections de la peau et des muqueuses, qui se caractérisent par des manifestations inesthétiques comme des rougeurs et des plaques de desquamation. Plusieurs pathologies sont regroupées sous la dénomination de dermatoses. On peut citer, à titre d'exemples non limitatifs, l'eczéma, la dermatite atopique, le psoriasis, les dermites séborrhéiques ou encore l'acné. Les dermatoses entraînent des altérations de la fonction barrière de l'épiderme se traduisant par une perméabilité de la peau, une sécheresse cutanée et une altération générale des fonctions protectrices de la peau vis-à-vis de l'environnement extérieur.

Le vieillissement naturel ou induit par exemple par l'exposition au soleil, des traitements médicamenteux, entraîne également ce type morphologique cutané avec altération de la fonction barrière de l'épiderme.

Ainsi l'invention concerne les applications d'un composé ou d'un sel de ce composé, ledit composé étant choisi parmi la vescalagine et la castalagine ainsi qu'un extrait de Lythrum salicaria L. contenant de l'acide gallique, de la vescalagine et de castalagine.

La structure de ces composés est ci-après reproduite :
L'acide gallique : 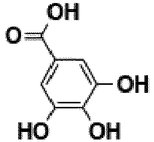
La vescalagine : 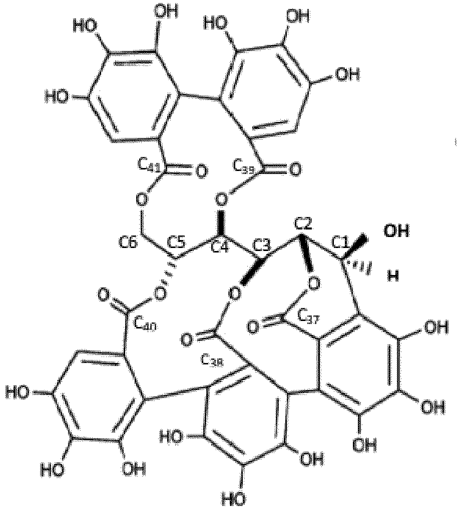
La castalagine :

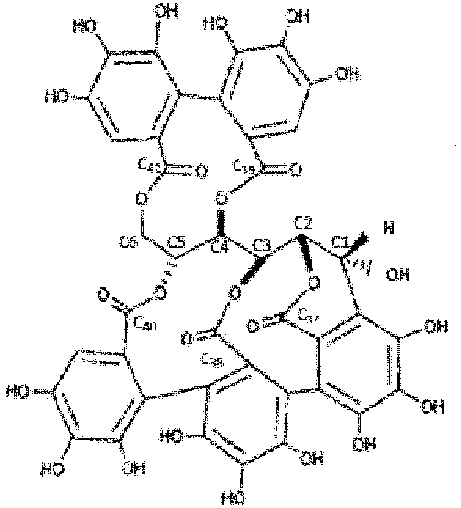

Un composé ci-dessus peut posséder un ou plusieurs centre(s) asymétrique(s). Selon l'invention, le composé peut être sous une forme optiquement active ou sous forme de son mélange racémique.

Certains composés de l'invention peuvent être obtenus à partir de plantes, par extraction et sont donc aisément accessibles. Ils peuvent aussi être utilisés selon la présente invention sous forme d'extraits de Lythrum salicaria
Selon l'invention, le composé peut se présenter sous forme salifiée, ainsi elle s'étend à tout sel dudit composé. Ce sel est avantageusement acceptable du point de vue cosmétique ou pharmaceutique. Un sel d'un composé de l'invention peut être un sel d'addition d'acide, ledit acide étant de préférence choisi parmi les acides chlorhydrique, bromhydrique, sulfurique phosphorique, acétique, trifluoacétique, lactique, pyruvique, malonique, succinique, glutarique, fumarique, tartrique, maléique, citrique, ascorbique, méthane- ou éthane-sulfonique, et camphorique. Il peut aussi être un sel d'addition de base, cette dernière étant préférentiellement choisie parmi l'hydroxyde de sodium ou de potassium, la triéthylamine ou la tertiobutylamine.

L'invention concerne les applications cosmétiques d'un composé ou d'un sel de ce composé, tels que définis ci-dessus, mais aussi le mélange de composés et/ou sels et/ou dérivés ainsi définis. Ainsi l'invention concerne leurs utilisations pour stimuler ou réparer la fonction barrière de l'épiderme. Plus particulièrement, elle vise leurs utilisations comme ingrédient actif dans une composition cosmétique pour stimuler ou réparer la fonction barrière de l'épiderme.

A ce titre, la concentration dudit composé, sel ou dérivé ou de leurs mélanges varie de 0,001% à 5% en poids par rapport au poids total de la composition, de préférence 0,01% à 5%, voire 0,05% à 5%, en poids par rapport au poids total de la composition.

L'invention a de plus trait aux applications pharmaceutiques, y compris vétérinaires, d'un composé, sel ou dérivé tel que défini ci-dessus. Ainsi, il trouve un intérêt particulier lorsqu'il est utilisé dans la prévention ou le traitement des lésions provoquées dans les parois d'organes par des pathologies, comme la maladie de Crohn, en particulier dans la paroi intestinale. Il est aussi avantageusement destiné à la prévention ou le traitement des lésions associées à des dermatoses comme la dermatite atopique, l'eczéma, le psoriasis, les dermites séborrhéiques et l'acné.

Elle concerne ainsi une composition pharmaceutique destinée à prévenir ou traiter les lésions provoquées par des pathologies, comme la maladie de Crohn et contenant une quantité efficace d'un composé de l'invention, ou d'un sel de ce composé, d'un dérivé de ce composé ou d'un sel d'un dérivé, tel que définis précédemment, et un véhicule acceptable du point de vue pharmaceutique.

Undit sel est compatible avec un usage pharmaceutique et de préférence un sel d'addition d'acides chlorhydrique, bromhydrique, sulfurique phosphorique, acétique, trifluoacétique, lactique, pyruvique, malonique, succinique, glutarique, fumarique, tartrique, maléique, citrique, ascorbique, méthane- ou éthane-sulfonique, ou camphorique, un sel d'addition de bases choisies parmi l'hydroxyde de sodium ou de potassium, la triéthylamine ou la tertiobutylamine.

Dans cette indication précise le composé, son sel ou la composition le ou les comprenant est destiné à une administration par voie orale.

Une composition pharmaceutique de l'invention répond en outre avantageusement aux caractéristiques ci-après, considérées seules ou en combinaison.
- le composé est sous forme de poudre, sous forme supportée par absorption sur un polymère organique en poudre tels que bentonite ou talc, ou sous forme encapsulée ;
- le composé est encapsulé dans des microsphères, des liposomes, des glycosphères, des chylomicrons, des macro-, micro-, ou nanoparticules, ou des macro-, micro- ou nanocapsules ;
- la composition contient un principe actif efficace dans les inflammations chroniques, tel qu'un principe actif choisi parmi des mucopolysaccharides, des vitamines, des céramides et des huiles végétales ;
- la composition comporte au moins un agent actif autre qu'un composé de l'invention pour exercer au moins une action complémentaire ou synergique.

Selon un aspect de l'invention, que ce soit pour des applications cosmétiques ou pharmaceutiques, dont vétérinaires, le composé, son sel ou dérivé et leurs mélanges sont utilisés sous forme d'extrait de plante, sans purification complète.

On entend par « extrait de plante », un extrait ou un mélange d'extraits de plantes des familles listées précédemment. Il s'agit plus spécialement d'un extrait ou d'un mélange d'extrait de cellules de ces familles. Ce matériel cellulaire peut être obtenu par culture *in vitro* ou *in vivo.* Par culture *in vitro,* on entend l'ensemble des techniques connues de l'homme du métier qui permettent de manière artificielle l'obtention d'un végétal ou d'une partie d'un végétal. Ainsi, l'extrait peut être un extrait ou un mélange d'extraits d'organe (racine, tige, feuille, écorce, fleur), voire de cellules d'organe, d'au moins une plante des familles précités, ou encore un extrait de cellules indifférenciées d'au moins une telle plante.

Un extrait selon l'invention peut être obtenu par toute méthode d'extraction ou de purification connue de l'homme du métier. On peut en particulier citer les procédés d'extraction solide-liquide en milieux alcooliques (notamment méthanoliques, éthanoliques), aqueux, ainsi qu'en milieux utilisant des solvants tels que les cétones, les esters, les éthers, les polyols, les solvants chlorés et les mélanges d'au moins deux des solvants précités, comme les milieux hydroalcooliques.

Avantageusement, un extrait est obtenu par extraction d'une famille de plantes précitées en milieu hydroalcoolique, notamment une solution aqueuse d'éthanol. Selon cette variante, la concentration de l'éthanol varie de préférence d'environ 20 à environ 40% (v/v), mieux encore elle est d'environ 30% (v/v).

Les méthodes alternatives aux solvants peuvent aussi être envisagées comme le CO₂ supercritique, les micro-ondes...

Ces modes de préparation font partie intégrante de l'invention.

Ces extraits peuvent être utilisés tels quels sous forme liquide ou en poudre, non purifiés ou purifiés.

Si l'extrait est en poudre, son séchage peut être conduit par toute technique bien connue de l'homme du métier. A titre d'exemple, l'extrait peut être séché par atomisation, évaporation ou lyophilisation. La poudre ainsi obtenue peut être encapsulée dans des liposomes ou autres vecteurs et supports pour une meilleure homogénéité de la composition et une meilleure diffusion du principe actif, notamment sur la peau.

Lorsque la plante utilisée selon l'invention est la salicaire, l'extrait est de préférence obtenu à partir des sommités fleuries. Les auteurs ont observé que les extraits obtenus par macération hydroalcoolique des parties aériennes de salicaire présentent une forte activité biologique. Ceci quel que soit le pourcentage d'éthanol dans le solvant d'extraction. Des résultats similaires sont observés dans l'eau ou de l'éthanol.

Les applications selon l'invention s'étendent au traitement des phanères et des muqueuses. Par phanères selon l'invention, on inclut notamment les ongles et le système pileux, en particulier les cheveux. Par muqueuses, on entend les tissus de revêtement des cavités anatomiques, qui se raccordent à la peau par des orifices naturels, comme la bouche, l'estomac, l'intestin, ainsi que ceux de cavités naturelles externes comme les narines, les oreilles.

Ainsi, la présente invention vise aussi l'utilisation cosmétique d'un composé ou d'un sel dudit composé, ou d'un mélange dedits composés et/ou de leurs sels, pour protéger la peau, les phanères et les muqueuses des agents extérieurs physiques, chimiques ou biologiques, et/ou pour améliorer et/ou renforcer l'hydratation de la peau et/ou renforcer les phanères et les muqueuses.

Elle concerne aussi un procédé de traitement cosmétique, non thérapeutique, de lutte contre la sécheresse d'une peau ou de traitement cosmétique d'une peau sèche, ledit procédé comprenant une étape selon laquelle on applique au moins un composé, sel ou dérivé, ou leurs mélanges tels que définis précédemment, sur la peau.

L'invention porte aussi sur un procédé de traitement cosmétique, non thérapeutique, anti-âge de la peau ou de traitement cosmétique, non thérapeutique, d'une peau âgée, comprenant une étape selon laquelle on applique au moins un composé, sel ou dérivé, ou leurs mélanges tels que définis précédemment, synthétique ou extrait d'une plante sur la peau. Ce traitement permet de retarder les phénomènes de vieillissement de la peau, mais aussi de réparer une peau âgée.

Dans toute application cosmétique de l'invention, un composé, sel ou dérivé, ou leurs mélanges tels que définis précédemment, peut être associé à un autre ou d'autres agents pro-différenciants de la peau, comme le calcium, la vitamine D et leurs dérivés.

Selon un autre aspect de l'invention, une composition selon l'invention se présente sous forme de gélules, crème, gel, lotion, lait, émulsion H/E ou E/H, solution, onguent, huile corporelle, shampoing, savon, bâton protecteur des lèvres, bâton et crayon pour maquillage.

Sous la forme de gel, la composition peut comprendre des excipients appropriés, tels que les esters de cellulose, ou d'autres agents gélifiants, tels que le polymère carboxylique « Carbopol® » et la gomme de guar, par exemple.

Sous la forme d'émulsions, les compositions selon l'invention jouissent d'une bonne stabilité et peuvent être conservées pendant le temps nécessaire pour l'utilisation à des températures comprises entre 0 et 50°C, sans qu'il y ait sédimentation des constituants ou séparation des phases.

Selon un autre aspect de l'invention, dans la composition, un composé, sel ou dérivé, ou leurs mélanges tels que définis précédemment, est mis en place dans des moyens d'encapsulation choisis dans le groupe formé par des microsphères, des liposomes, des glycosphères, des chylomicrons, des macro-, micro- et nanoparticules, des macro-, micro- et nanocapsules.

Selon un autre aspect de l'invention, dans la composition, un composé, sel ou dérivé, ou leurs mélanges tels que définis précédemment, est absorbé ou adsorbé sur des polymères organiques poudreux, des talcs, de la bentonite ou d'autres supports minéraux en poudre bien connus de l'homme du métier.

Selon un autre aspect de l'invention, dans la composition, un composé, sel ou dérivé, ou leurs mélanges tels que définis précédemment, constitue de 0,001% à 5% en poids par rapport au poids total de la composition, de préférence 0,01% à 5%, voire 0,05% à 5%.

Selon un autre aspect de l'invention, dans la composition, un composé, sel ou dérivé, ou leurs mélanges tels que définis précédemment, est sous forme encapsulée et constitue, de préférence de 0,05 à 5% en poids par rapport au poids total de la composition.

Ainsi, la présente invention a pour objet une utilisation cosmétique d'au moins un composé, sel ou dérivé, ou leurs mélanges tels que définis précédemment, pour apaiser les peaux sèches à tendance atopique et/ou pour améliorer et/ou renforcer l'hydratation de la peau. En particulier, elle concerne les utilisations d'au moins un composé, sel ou dérivé, ou leurs mélanges tels que définis précédemment, comme ingrédient actif dans une composition cosmétique destinée à être appliquée sur une peau sèche à tendance atopique, à la lutte contre le vieillissement de la peau tel que le vieillissement lié à l'âge et/ou le photo-vieillissement, à la lutte contre les démangeaisons et contre le prurit, à l'hydratation et à la protection contre les agressions externes liées à la pollution et au stress.

Dans la visée thérapeutique de l'invention, un composé, sel ou dérivé, ou leurs mélanges tels que définis précédemment, peut être administré par voie topique mais il peut aussi être administré par voie orale. Il peut être utilisé tel quel sous forme liquide ou en poudre, non purifié ou purifié.

Un composé, sel ou dérivé, ou leurs mélanges tels que définis précédemment, peut, dans les compositions selon l'invention, être associé à d'autres composés venant compléter l'effet voire synergiser cet effet.

Son activité protectrice vis-à-vis des radicaux libres et des UV est intéressante dans le domaine capillaire, notamment dans le cas d'association avec des substances facilitant le bon état du cuir chevelu et du cheveu. On peut citer les associations avec des mucopolysaccharides, des minéraux, des vitamines, des céramides, des huiles végétales, des substances antiradicalaires, des filtres U.V., des acides de fleurs ou de fruits.

De même, l'activité réparatrice d'un composé, sel ou dérivé, ou leurs mélanges tels que définis précédemment, est particulièrement intéressante, quand il est associé avec des substances ayant un effet cicatrisant comme des protéines, l'acide hyaluronique, des acides aminés, ou avec des substances anti-inflammatoires, anti-âge, après-solaire anti-acné ou anti-dermatose.

Ainsi, les compositions de l'invention sont tout particulièrement adaptées à une application topique pour prévenir et/ou traiter de nombreuses altérations cutanées, notamment comme agent réparateur et/ou protecteur de la peau et du système pileux comme les cheveux, pour lutter contre les agressions externes liées à la pollution, au soleil, au stress oxydatif, au vieillissement et aux pathologies cutanées entraînant un dysfonctionnement de l'homéostasie de l'épiderme ou des cheveux.

Ces compositions cosmétiques peuvent aussi prendre la forme de lotion ou solution dans laquelle les dérivés selon l'invention sont sous forme encapsulée, par exemple dans des microsphères. Ces microsphères peuvent, par exemple, être constituées de corps gras, d'agar et d'eau. Les agents actifs peuvent être aussi incorporés dans des vecteurs de type liposomes, glycosphères, dans des chylomicrons, des macro-, micro-, nanoparticules ainsi que les macro-, micro- et nanocapsules et aussi être adsorbés sur des polymères organiques poudreux, les talcs, bentonites et autres supports minéraux.

Pour la préparation des compositions selon l'invention, un composé, sel ou dérivé, ou leurs mélanges tels que définis précédemment, peut être mélangé aux excipients généralement employés en cosmétique. Les compositions cosmétiques de l'invention peuvent donc contenir des additifs ou des adjuvants usuels en cosmétologie, comme par exemple des agents antibactériens ou des parfums mais aussi des lipides d'extraction et/ou de synthèse, des polymères gélifiants et viscosants, des tensio-actifs, des émulsifiants, des actifs hydro- ou lipo-solubles, des extraits de plantes, des extraits tissulaires, des extraits marins, ou des actifs de synthèse.

Une composition dermocosmétique ou pharmaceutique de l'invention comprend tous les produits de soin du corps et de la peau, y compris les produits solaires, protecteurs et bronzants, les produits anti-âge, anti-séborrhéiques, toniques, les produits assurant l'amélioration de l'aspect de la peau y compris le traitement acnéique, le traitement des rougeurs cutanées, le traitement du cuir chevelu et celui de la chute des cheveux.

Les compositions cosmétiques de la présente invention peuvent aussi comprendre d'autres agents actifs complémentaires choisis pour leur action, par exemple pour la protection solaire, l'effet anti-rides, l'activité antiradicalaire et antioxydante, l'activité anti-irritante, la nutrition cellulaire, la respiration cellulaire, l'hydratation et la régénération cellulaire, les traitements anti-séborrhéïques, ainsi que d'autres agents actifs ayant une action sur la tonicité cutanée et la protection du cheveu.

Les compositions cosmétiques de la présente invention sont, de préférence, à utiliser quotidiennement en les appliquant une ou plusieurs fois par jour.

Les compositions cosmétiques de la présente invention sont très bien tolérées, elles ne présentent aucune phototoxicité et leur application sur la peau, pour des périodes de temps prolongées, n'implique aucun effet systémique.

L'invention concerne aussi l'utilisation d'un composé, d'un sel de ce composé, d'un dérivé de ce composé, d'un sel de ce dérivé, ou de leurs mélanges tels que définis précédemment, pour la préparation de compositions pharmaceutiques présentant une activité anti-inflammatoire et/ou dermoprotectrice. Ces compositions sont utiles pour prévenir et/ou traiter notamment des maladies dermatologiques liées à des activités séborrhéiques, acnéiques, inflammatoires et immunologiques.

La présente invention est maintenant illustrée par les exemples donnés ci-après. Ils font référence aux figures 1 à 11, selon lesquelles :
La figure 1 représente le chromatogramme de l'extrait de salicaire obtenu en HPLC semi-préparative dans les conditions décrites à l'exemple 2, représentant la concentration (exprimée en unités d'absorbance) des constituants d'un extrait de salicaire en fonction du temps de rétention (en minutes).
Les figures 2, 3 et 4 représentent les effets d'un extrait de salicaire et de la vescalagine sur la libération de trois médiateurs, MMP-1 (figure 2), MMP-3 (figure 3) et procollagène 1 (figure 4) par des peaux reconstruites après 9 jours en milieu déplété.
La figure 5 représente l'influence d'un extrait de salicaire, EX10MF1046, sur l'expression de différents marqueurs de la différenciation épidermique, par les cultures de kératinocytes à l'état basal, évaluée par RT-qPCR.
La figure 6 présente les images numériques de coupe d'épidermes reconstruits (RHE) traités par un témoin, une référence et l'extrait de salicaire, EX10MF1046, à 20 µg/ml, et leurs effets sur l'expression de marqueurs de la différenciation épidermique.
La figure 7 représente l'influence de l'extrait de salicaire, EX10MF1046, sur l'expression de différents marqueurs de la différenciation épidermique, par les cultures de kératinocytes stimulées ou non par un mélange de cytokines IL-17, OSM et TNFα (mix-PSO), évaluée par RT-qPCR.
La figure 8 présente les images numériques de coupes d'épidermes reconstruits stimulés par un mélange de cytokines IL-17, OSM, TNFα traités par un témoin, une référence (JAK inhibitor 1) et l'extrait de salicaire, EX10MF1046 à 20 µg/ml, et leurs effets sur l'expression du marqueur de différenciation épidermique KRT10.
La figure 9 représente l'influence de l'extrait de salicaire, EX10MF1046, sur l'expression de différentes chimiokines induites par l'inflammation, par les cultures de kératinocytes stimulées ou non par un mélange de cytokines IL-17, OSM et TNFα (mix-PSO), évaluée par RT-qPCR.
La figure 10 représente l'influence de l'extrait de salicaire, EX10MF1046, sur la libération de l'IL-8 par des épidermes reconstruits (RHE à J13) stimulés par le mélange de cytokines IL-17, OSM et TNFα (mix-PSO) pendant 48h, observés à la figure 7.
La figure 11 présente un schéma d'une boucle inflammatoire chronique de la peau (par exemple, psoriasis) illustrant le stade d'intervention de l'extrait de salicaire, EX10MF1046, dans cette boucle.

### Exemple 1 : Obtention d'extraits de salicaire (Lythrum salicaria L) comprenant un composé de l'invention

Différents extraits de salicaire (EX3MF1046, EX4MF1046, EX1MF1046 et EX2MF1046) ont été obtenus dans le présent exemple, selon un protocole général décrit ci-après et dont les conditions spécifiques à chaque extrait sont spécifiées dans le tableau 1.

### Protocole général :

Une masse de parties aériennes de salicaire, préalablement séchées, est broyée et mise à macérer pendant 24 heures dans un solvant de macération. Pendant toute la durée de la macération, le mélange a été maintenu sous agitation, à 20°C. Après macération, le résidu de plante est séparé de l'extrait par clarification et filtration.

L'extrait est ensuite directement concentré par évaporation sous vide, puis séché par atomisation et obtenu sous forme d'une poudre, à l'exception des extraits EX10MF1046 et EX13MF1046 qui, avant concentration, sont mis au contact de charbon actif (0,5kg), laissés sous agitation pendant une heure puis séparés du charbon actif par filtration.

**Tableau 1**

| Référence de l'extrait | Masse de salicaire traitée | Solution de macération | | Extrait |
|---|---|---|---|---|
| | | Masse | Solvant | Masse |
| EX3MF1046 | 5kg | 50kg | Ethanol dans eau 30% (v/v) | 550g |
| EX10MF1046 | 5kg | 50kg | Ethanol dans eau 30% (v/v) | 300g |
| EX4MF1046 | 50g | 500g | Eau | 3g |
| EX1MF1046 | 50g | 500g | Ethanol dans eau 90% (v/v) | 2g |
| EX2MF1046 | 50g | 500g | Ethanol dans eau 90% (v/v) | 4g |
| EX13MF1046 | 25g | 500g | Acétate d'éthyle | 300mg |

### Exemple 2 : Isolement d'un composé de l'invention à partir d'un extrait de salicaire et caractérisation

### 1) Matériels et méthodes

### Extrait

Un extrait de salicaire (*Lythrum* salicaria L.) est obtenu par macération des sommités fleuries dans une solution hydroalcoolique (éthanol 30%) pendant 24 heures. L'extrait a ensuite été concentré par évaporation puis lyophilisé.

### Purification

Une première étape de purification est réalisée par extraction sur phase solide (SPE). Le support choisi est une silice greffée C18. L'extrait est déposé en solution dans une solution hydroalcoolique contenant 20 % de méthanol et 0,5 % d'acide formique. L'élution est réalisé avec le même solvant. La fraction obtenue est concentrée par évaporation puis lyophilisée.

Une seconde étape de purification est réalisée par HPLC semi-préparative sur une colonne Synergi Fusion (Phenomenex) de dimensions 250x10mm. L'élution est assurée par un gradient acide formique 0,5 % et méthanol.

### Caractérisation

L'acide gallique a été identifié par comparaison des caractéristiques physicochimiques (rétention, spectre d'absorption ultraviolet et spectre de masse) du composé présent dans l'extrait à celles d'un échantillon commercial d'acide gallique (Sigma Aldrich - référence 27645).

Les autres composés purifiés ont été caractérisés par Résonance Magnétique Nucléaire (RMN) du proton (400 MHz) et du carbone 13 (100 MHz).

### 2) Résultats

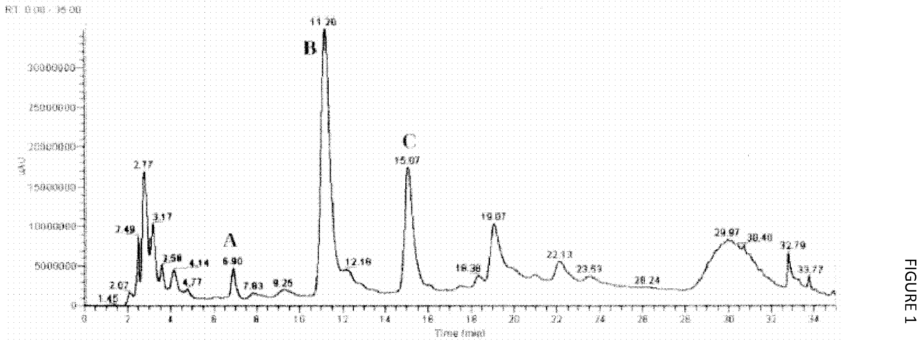

La figure 1 représente le chromatogramme de l'extrait de salicaire.

Le composé A présente un maxima d'absorbance UV à 269 nm et une masse molaire monoisotopique de 170 g/mol. Son temps de rétention dans les conditions HPLC utilisées est de 6,90 minutes. L'échantillon commercial d'acide gallique présente exactement les mêmes caractéristiques physico-chimiques. Ces éléments permettent d'identifier le composé A comme étant l'acide gallique.

Les tableaux 2 et 3 ci-dessous rassemblent les déplacements chimiques du composé B, respectivement en RMN du carbone 13 et en RMN du proton. L'ensemble de ces déplacements chimiques permettent d'identifier le composé B comme étant la vescalagine.

**Tableau 2**

| Pic B | | | |
|---|---|---|---|
| Signal | Déplacement chimique (ppm) | Attribution | Caractéristique |
| 1 | 65,15 | C6 | C sucre |
| 2 | 65,37 | C1 | C sucre |
| 3 | 68,29 | C3 | C sucre |
| 4 | 69,17 | C4 | C sucre |
| 5 | 71,03 | C5 | C sucre |
| 6 | 77,61 | C2 | C sucre |
| 7-36 | 107,26-147,30 | 30 carbones | C aromatiques |
| 37 | 165,37 | C=O | Fonction ester |
| 38 | 165,49 | C=O | Fonction ester |
| 39 | 166,50 | C=O | Fonction ester |
| 40 | 167,23 | C=O | Fonction ester |
| 41 | 169,27 | C=O | Fonction ester |

**Tableau 3**

| Pic B | | |
|---|---|---|
| Proton | Déplacement chimique δ (ppm) | Données spectrales |
| H1 | 4,87 | d ; 1 ; J=2 Hz |
| H2+ H4 | 5,19-5,23 | m ; 2H |
| H3 | 4,56 | dd ; 1H ; J=0,8 et 6,8 Hz |
| H5 | 5,65 | d ; 1H ; J=6,4 Hz |
| H6 | 5,08 | dd ; 1H ; J=2,8 et 13,2 Hz |
| H6' | 4,01 | d ; 1H ; J=12,8 Hz |
| H aromatiques | 6,61 ; 6,76 ; 6,77 | s ; 1H |

Les tableaux 4 et 5 rassemblent les déplacements chimiques du composé C, respectivement en RMN du carbone 13 et en RMN du proton. L'ensemble de ces déplacements chimiques permettent d'identifier le composé C comme étant la castalagine.

**Tableau 4**

| Pic C | | | |
|---|---|---|---|
| Signal | Déplacement chimique (ppm) | Attribution | Caractéristique |
| 1 | 64,65 | C6 | C sucre |
| 2 | 65,62 | C1 | C sucre |
| 3 | 66,38 | C3 | C sucre |
| 4 | 68,51 | C4 | C sucre |
| 5 | 70,50 | C5 | C sucre |
| 6 | 73,36 | C2 | C sucre |
| 7-36 | 106,85-145,99 | 30 carbones | C aromatiques |
| 37 | 164,04 | C=O | Fonction ester |
| 38 | 164,98 | C=O | Fonction ester |
| 39 | 165,99 | C=O | Fonction ester |
| 40 | 166,55 | C=O | Fonction ester |
| 41 | 168,58 | C=O | Fonction ester |

**Tableau 5**

| Pic C | | |
|---|---|---|
| Proton | Déplacement chimique δ (ppm) | Données spectrales |
| H1 | 5,71 | d ; 1H ; J=4,40 Hz |
| H2+ H3 | 5,03-5,05 | m ; 2H ; J=5,6 Hz |
| H4 | 5,25 | t; 1H ; J=7,20 Hz |
| H5 | 5,62 | dd ; 1H ; J=1,60 et 8,00 Hz |
| H6 | 5,11 | dd ; 1H ; J=2,40 et 12,80 Hz |
| H6' | 4,00 | d ; 1H ; J=12,80 Hz |
| H aromatiques | 6,63 ; 6,78 ; 6,79 | s ; 1H |

### Exemple 3 : Evaluation de composés de l'invention dans des primocultures de kératinocytes épidermiques humains normaux (NHEK)

### 1) Matériels et méthodes

### Cellules

Kératinocytes épidermiques humains normaux (NHEK) ; référence BIOalternatives K341, utilisés au troisième passage.
Conditions de culture : 37°C, 5% CO₂
Milieu de culture : KeratinocyteSFM complémenté avec facteur de croissance épidermique (EGF) 0,25 ng/ml, extrait pituitaire (EP) 25 µg/ml et gentamycine 25 µg/ml.
Milieu d'essai : KeratinocyteSFM complémenté avec gentamycine 25 µg/ml.

### Composés testés

Après évaluation de cytotoxicité préalable, les composés acide gallique, vescalagine, castalagine et salicarinine A sont testés aux concentrations suivantes :
Acide gallique, 0,0033 ; 0,01 et 0,03 mg/ml
Vescalagine, 0,0011 ; 0,003 et 0,01 mg/ml
Castalagine, 0,0011 ; 0,003 et 0,01 mg/ml
Salicarinine A, 0,0011 ; 0,003 et 0,01 mg/ml

### Immunomarquages in situ

Les kératinocytes ont été ensemencés et cultivés à confluence en plaques 96 puits puis le milieu a été remplacé par du milieu d'essai contenant ou non (témoin) l'extrait testé ou la référence (CaCl₂ à 1,5 mM) et les cellules ont été incubées pendant 72 heures. Toutes les conditions expérimentales ont été réalisées en n=3, pour chaque marqueur.

Après incubation, le milieu d'essai est éliminé et les cellules sont rincées, fixées et perméabilisées. Les cellules sont marquées avec les anticorps primaires dirigés contre les protéines d'intérêt (TGK). Ces anticorps ont été révélés par un anticorps secondaire couplé à un fluorochrome (GAMAlexa 488). En parallèle, les noyaux des cellules sont colorés par le Hoechst 33258 (bisbenzimide). L'acquisition des images est réalisée avec un système d'imagerie à haute résolution, INCell Analyzer™1000 (GE Healthcare). Pour chaque puits, 5 saisies d'images numérisées sont effectuées. Les marquages sont quantifiés par mesure de l'intensité de fluorescence des protéines rapportée au nombre de noyaux identifiés par le Hoechst (intégration des données numériques par le logiciel Developer Toolbox 1.5, GE Healthcare).

### 2) Résultats

Le tableau 6 illustre le niveau d'expression de la TGK via le marquage fluorescent des NHEK pour différentes concentrations de vescalagine, castalagine et salicarinine A.

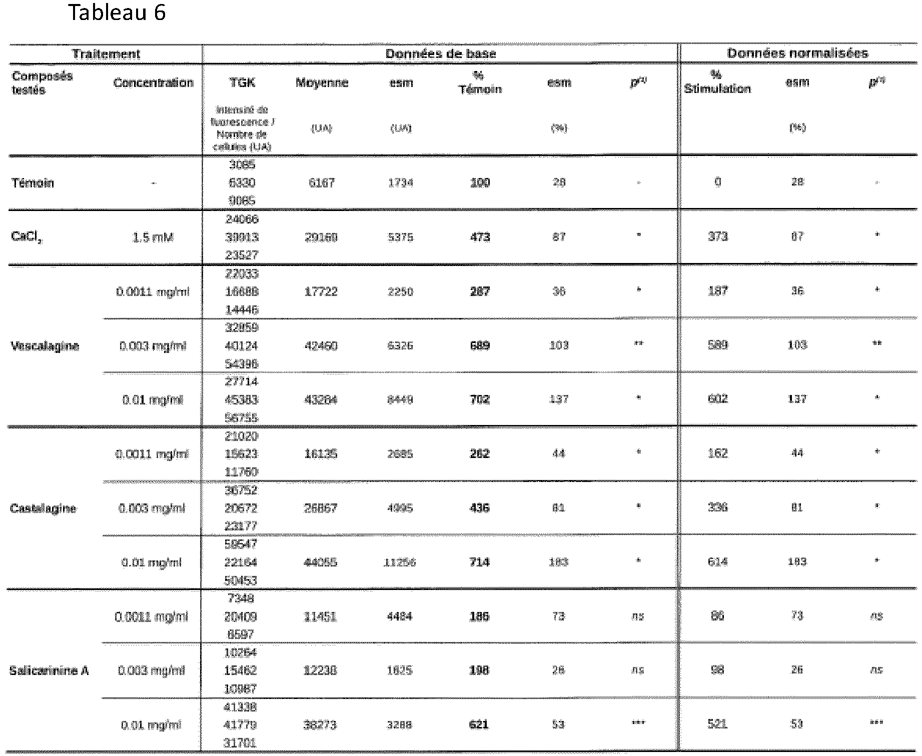

Le tableau 7 illustre le niveau d'expression de la TGK via le marquage fluorescent des NHEK pour différentes concentrations d'acide gallique.

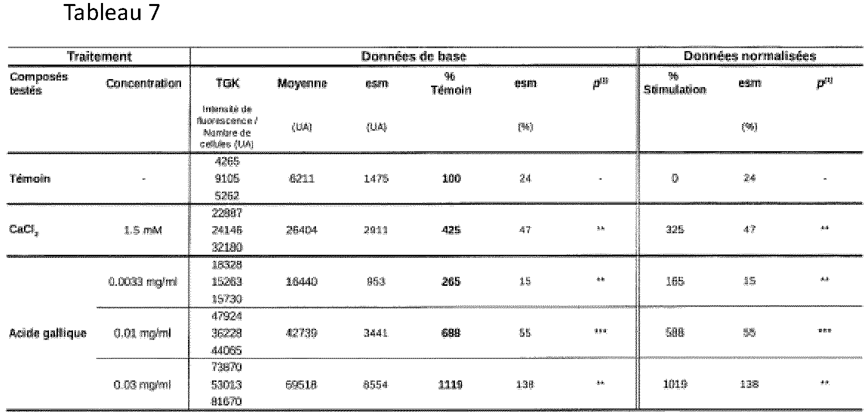

Dans les tableaux 6 et 7, le seuil de significativité statistique est le suivant :
Ns : > 0,05, Non significatif
* : 0,01 à 0,05, Significatif
** : 0,001 à 0,01, Très significatif
*** < 0,001, Extrêmement significatif

Ces résultats montrent que l'acide gallique, la vescalagine, la castalagine et la salicarinine A induisent de façon dose-dépendante l'expression de TGK dans les cultures de NHEK. L'activité est supérieure à celle de la référence chlorure de calcium, dès la concentration de 10 µg/ml.

### Exemple 4 : Evaluation de composés de l'invention sur des peaux reconstruites

### 1) Matériels et méthodes

### Peaux reconstruites (PR)

Peaux humaines reconstruites à partir d'une culture de kératinocytes ensemencés sur une lattice de collagène contenant des fibroblastes.

Les kératinocytes sont cultivés à 37°C, 5% CO₂, à l'interface air-liquide dans un des milieux suivants :
Milieu de culture complet : Epilife et compléments (sans IGF-1) + CaCl₂ 1,5 mM + insuline bovine 5 µg/ml + vitamine C 50 µg/ml + KGF (facteur de croissance des kératinocytes) 3 ng/ml, ou
Milieu de culture déplété : Epilife et compléments (sans IGF-1 et sans hydrocortisone) + CaCl₂ 1,5 mM + vitamine C (50 µg/ml).

### Composés testés

Extrait de salicaire obtenu à l'exemple 2 testé à 30 µg/ml et 60 µg/ml
Vescalagine testée à 30 µg/ml et 60 µg/ml

### Description du test

Les cultures suivantes sont réalisées :
- Peaux reconstruites non traitées,
- Peaux reconstruites en milieu déplété,
- Peaux reconstruites en milieu déplété, traitées chacune par un composé testé ci-dessus, à une concentration de 30 µg/ml ou de 60 µg/ml.

Les peaux reconstruites sont placées lors du passage à l'interface air-liquide (J0) en plaque 6 puits en milieu de culture déplété contenant ou non (condition témoin carencé) les composés testés. Les peaux reconstruites sont ensuite été cultivées pendant 9 jours avec renouvellement du milieu de culture contenant ou non les composés testés tous les 2 à 3 jours de J0 à J5, puis tous les jours entre J5 et J9. En parallèle, une culture des peaux reconstruites en milieu complet a été réalisée, à titre de témoin.

Toutes les conditions expérimentales ont été réalisées en n=4.

L'évaluation des composés testés est effectuée :
✔ Par analyse histologique, avec observation de l'épaisseur épidermique en histologie après coloration HES (Hématéine/Eosine/Safran),
✔ Par RT-qPCR, par analyse de l'expression de 16 marqueurs (dont 2 gènes de référence) sélectionnés pour leur importance dans le vieillissement cutané, sur les ARNm extraits de peaux reconstruites pour chaque traitement, et
✔ Par quantification de la libération de MMP-1, MMP-3 et procollagène I dans les sous-nageants de culture à J9, par dosage ELISA, à l'aide de kits de dosage ELISA utilisés selon les instructions du fournisseur.

### 2) Résultats

### Analyse HES

### - Peaux reconstruites en milieu complet, non traitées :

Elles présentent une histologie normale avec la présence des structures caractéristiques d'une peau reconstruite :
Un compartiment dermique composé de fibroblastes dans une matrice de collagène
Un compartiment épidermique avec les différentes couches de cellules :
   Une couche basale (une couche de cellules en palissade)
   Une couche épineuse (4 à 5 couches de cellules)
   Une couche granuleuse (une couche de cellules présentant des grains de kératohyaline), et
   Une couche cornée (cellules mortes anucléées).

### - Peaux reconstruites en milieu déplété, non traitées :

Elles présentent une histologie modifiée en comparaison avec les peaux reconstruites non traitées avec :
Déstructuration générale de l'épiderme avec :
   Perte de la couche basale
   Couche granuleuse moins importante (diminution du nombre de grains de kératohyaline)
   Couche cornée épaisse et parakératose
Perte de la cohésion épiderme/derme représentait par le « glissement » ou le décrochage de l'épiderme par rapport au derme
Aspect global des PR : épaisseur plus importante et longueur moins importante (ratatiné).

### - Peaux reconstruites en milieu déplété, traitées par l'extrait de salicaire à 30 µg_{/}ml et 60 µg_{/}ml :

Elles présentent une amélioration de leur histologie avec :
Récupération de certaines couches cellulaires :
   Une couche basale (une couche de cellules en palissade)
   Une couche granuleuse plus importante
Récupération de la cohésion épiderme/derme
Récupération des dimensions globales observées sur les PR non traitées.

### - Peaux reconstruites en milieu déplété, traitées par la vescalagine à 30 µg_{/}ml et 60 µg_{/}ml

Elles présentent une amélioration de leur histologie avec :
Récupération de certaines couches cellulaires :
   Une couche basale (une couche de cellules en palissade)
   Une couche granuleuse plus importante
Récupération partielle de la cohésion épiderme/derme
Récupération des dimensions globales observées sur les peaux reconstruites non traitées.

### RT-qPCR

Les résultats sont présentés dans les tableaux 8 (extrait de salicaire) et 9 (vescalagine) ci-dessous.

Dans ces tableaux, les cellules grisées des colonnes [% Témoin Moyenne HK] correspondent à un effet inhibiteur ou stimulant de l'extrait de salicaire ou de la vescalagine.

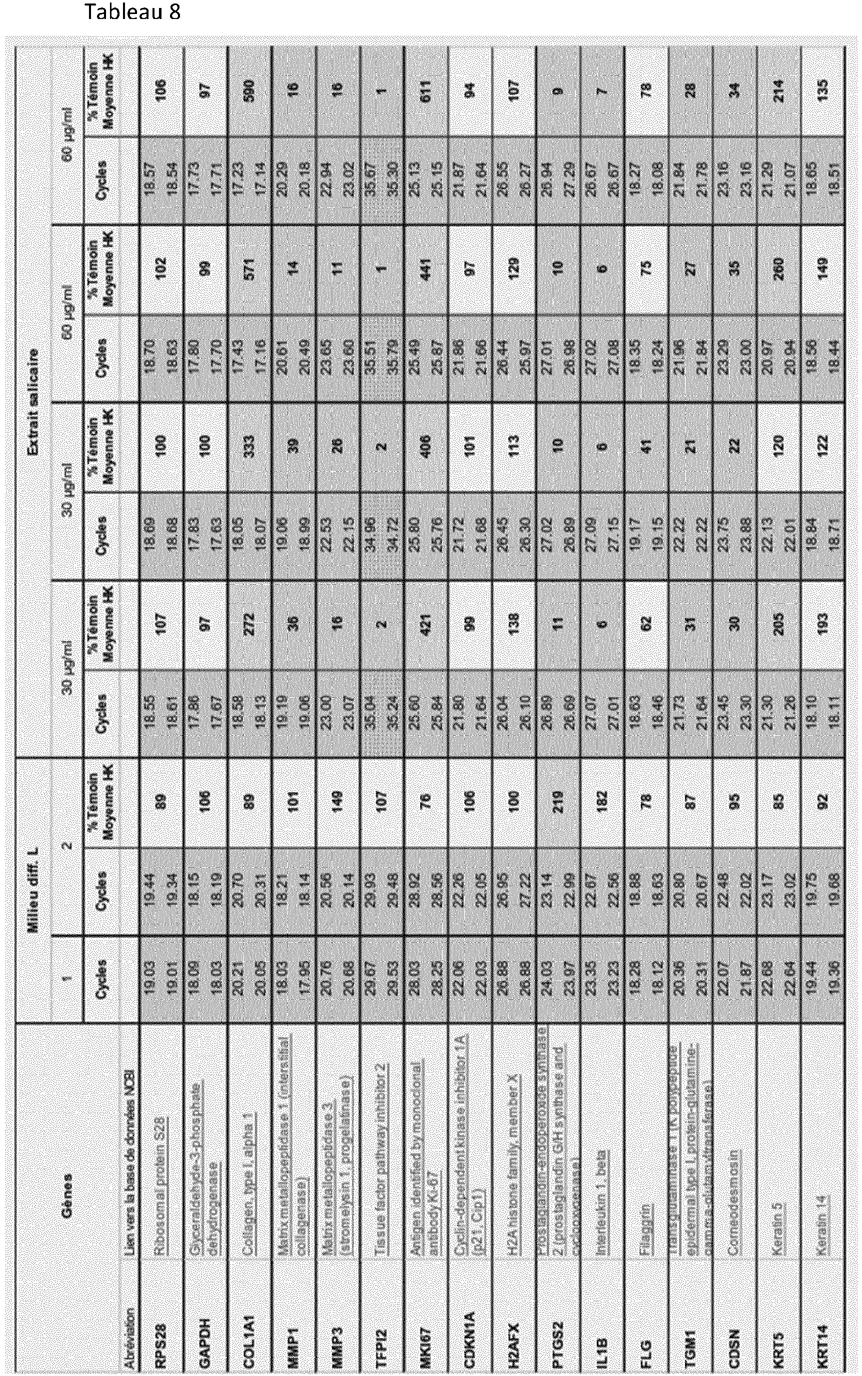

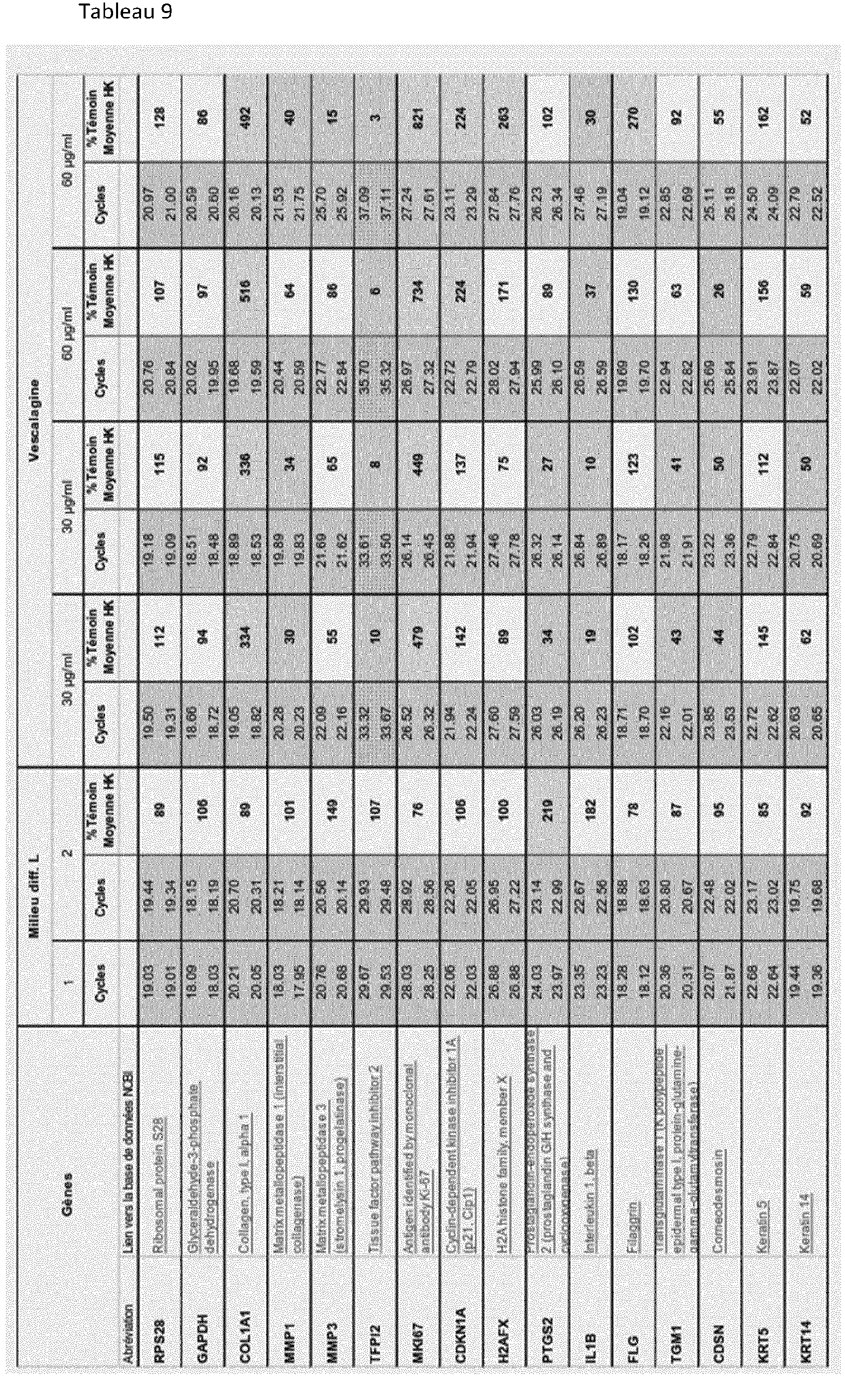

Les profils d'expression des gènes sélectionnés sont très différents entre les peaux reconstruites cultivées en milieu complet et celles cultivées en milieu déplété. Les peaux reconstruites en milieu déplété présentent une très forte augmentation de l'expression de marqueurs de la dégradation de la matrice (MMP1, MMP3, TFPI2), de marqueurs de l'inflammation (PTGS2, IL1B), et de marqueurs de la différenciation des kératinocytes (FLG, TGM1, CDSN). En parallèle, une très forte diminution de l'expression du marqueur du cycle cellulaire MKI67, de l'expression du marqueur de la matrice extracellulaire COL1A1 et des marqueurs des kératinocytes basaux, KRT5 et KRT14, est observée. Ces résultats sont attendus et valident l'essai.

L'extrait de salicaire, testé en systémique à 30 µg/ml et 60 µg/ml en milieu carencé a permis de compenser, de façon dose-dépendante, l'effet du milieu carencé avec notamment :
- un effet au niveau des marqueurs des cellules de la couche basale avec une augmentation de l'expression du marqueur de différenciation épidermique KRT5 (KRT14 dans une moindre mesure) et une augmentation du marqueur du cycle cellulaire MKI67. Ces modifications sont en corrélation avec les observations histologiques qui montrent la récupération d'une couche basale distincte suite au traitement ;
- un effet au niveau des marqueurs d'inflammation avec une diminution de l'expression de PTGS2 et d'IL1B;
- un effet au niveau des marqueurs plus tardifs de la différenciation des kératinocytes avec une diminution de l'expression TGM1 et CDSN. Ces résultats sont en corrélation avec les observations histologiques qui montrent une meilleure différenciation terminale des kératinocytes (couche granuleuse et couche cornée se rapprochant des peaux reconstruites « contrôles »);
- un effet au niveau des marqueurs impliqués dans la dégradation de la matrice extracellulaire avec une diminution de l'expression des gènes MMP1, MMP3 et TFPI2. Parallèlement, une augmentation de l'expression du marqueur de la matrice extracellulaire COL1A1 est observée.

La vescalagine, testée en systémique à 30 µg/ml et 60 µg/ml en milieu déplété a permis de compenser partiellement, de façon dose-dépendante, l'effet du milieu carencé avec notamment :
- un effet au niveau des marqueurs des cellules de la couche basale avec une augmentation du marqueur du cycle cellulaire MKI67. Ces modifications sont en corrélation avec les observations histologiques qui montrent la récupération d'une couche basale distincte suite au traitement ;
- un effet au niveau des marqueurs d'inflammation avec une diminution de l'expression d'IL1B et dans une moindre mesure PTGS2 ;
- un léger effet au niveau des marqueurs plus tardifs de la différenciation des kératinocytes avec une diminution de l'expression TGM1 et CDSN. Ces résultats sont en corrélation avec les observations histologiques qui montrent une meilleure différenciation terminale des kératinocytes (couche granuleuse et couche cornée se rapprochant des peaux reconstruites « contrôles ») ;
- un effet au niveau des marqueurs impliqués dans la dégradation de la matrice extracellulaire avec une diminution de l'expression des gènes MMP1, MMP3 et TFPI2. Parallèlement, une augmentation de l'expression du marqueur de la matrice extracellulaire COL1A1 est observée.

### ELISA pour dosages MMP-1, MMP-3 et procollagène I

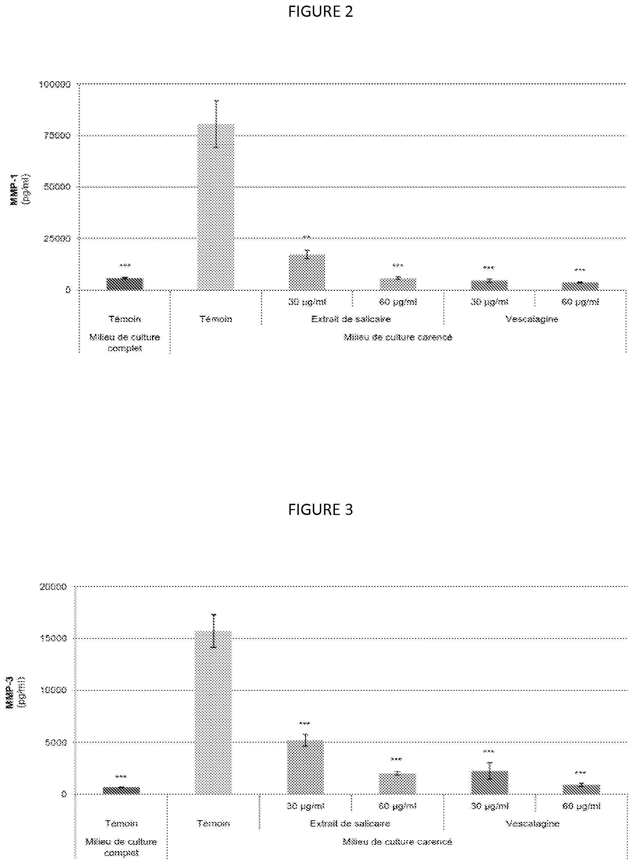

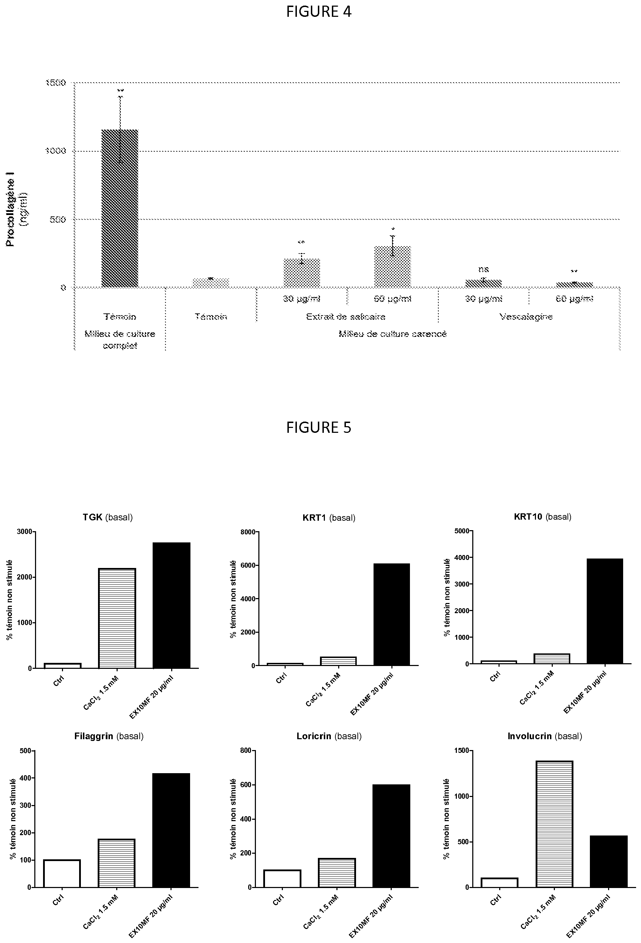

Les résultats des dosages dans les sous-nageants de culture sont présentés dans les figure 2 (MMP-1), figure 3 (MMP-3) et figure 4 (procollagène 1).

On observe pour les peaux reconstruites en milieu de culture déplété :
- Augmentation très importante de la libération de protéases impliquées dans la dégradation de la matrice extracellulaire, MMP-1 et MMP-3, et
- Diminution nette de la libération d'une protéine de la matrice extracellulaire, le procollagène I

Le traitement systémique par l'extrait de salicaire à 30 µg/ml et 60 µg/ml des peaux reconstruites en milieu de culture déplété induit une diminution importante et significative de la libération de MMP-1 et de MMP-3. Parallèlement, il stimule significativement la libération du procollagène I.

Le traitement systémique par la vescalagine à 30 µg/ml et 60 µg/ml des peaux reconstruites en milieu de culture déplété induit une diminution importante et significative de la libération de MMP-1 et de MMP-3. Aucune modification n'a été observée sur la libération du procollagène I.

Il ressort de cet exemple que l'extrait de salicaire et la vescalagine ont protégé les peaux reconstruites du vieillissement induit par le milieu de culture déplété (carencé).

### Exemple 5 : Evaluation d'extraits de salicaire dans des primocultures de kératinocytes épidermiques humains normaux (NHEK)

### 1) Matériels et méthodes

### Cellules

Kératinocytes épidermiques humains normaux (NHEK) ; référence BIOalternatives K341, utilisés au troisième passage.
Conditions de culture : 37°C, 5% CO₂
Milieu de culture : KeratinocyteSFM complémenté avec facteur de croissance épidermique (EGF) 0,25 ng/ml, extrait pituitaire (EP) 25 µg/ml et gentamycine 25 µg/ml.
Milieu d'essai : KeratinocyteSFM complémenté avec gentamycine 25 µg/ml.

### Extraits testés

Dans l'étape initiale de sélection d'un extrait, et après évaluation de cytotoxicité préalable, les extraits EX1MF1046, EX2MF1046, EX3MF1046, EX4MF1046 et EX13MF1046 de l'exemple 1, sont testés aux concentrations suivantes :
EX1MF1046, 0,005 mg/ml
EX2MF1046, 0,003 mg/ml
EX3MF1046, 0,005 mg/ml
EX4MF1046, 0,005 mg/ml
EX13MF1046, 0,005 mg/ml

L'étape ci-dessus a conduit à retenir les extraits EX3MF1046 et EX10MF1046 qui correspond à l'extrait EX3MF1046 filtré, pour les essais suivants. Ils sont évalués aux concentrations suivantes :
EX3MF1046, entre 0,0016 mg/ml et 0,02 mg/ml ; concentration non cytotoxique.
EX10MF1046, 0,02 mg/ml

### Immunomarquages in situ

Les kératinocytes ont été ensemencés et cultivés à confluence en plaques 96 puits puis le milieu a été remplacé par du milieu d'essai contenant ou non (témoin) l'extrait testé ou la référence (CaCl₂ à 1,5 mM) et les cellules ont été incubées pendant 72 heures. Toutes les conditions expérimentales ont été réalisées en n=3, pour chaque marqueur.

Après incubation, le milieu d'essai est éliminé et les cellules sont rincées, fixées et perméabilisées. Les cellules sont marquées avec les anticorps primaires dirigés contre les protéines d'intérêt (TGK, filaggrine et KRT10) ¹. Ces anticorps ont été révélés par un anticorps secondaire couplé à un fluorochrome (GAMAlexa 488). En parallèle, les noyaux des cellules sont colorés par le Hoechst 33258 (bisbenzimide). L'acquisition des images est réalisée avec un système d'imagerie à haute résolution, INCell Analyzer™1000 (GE Healthcare). Pour chaque puits, 5 saisies d'images numérisées sont effectuées. Les marquages sont quantifiés par mesure de l'intensité de fluorescence des protéines rapportée au nombre de noyaux identifiés par le Hoechst (intégration des données numériques par le logiciel Developer Toolbox 1.5, GE Healthcare).

Le screening des extraits n'a été réalisé que sur le paramètre TGK (tableau 10).

### Analyse des transcrit, RT-qPCR

Les kératinocytes ont été ensemencés et cultivés à confluence en plaques 24 puits puis le milieu a été remplacé par du milieu d'essai contenant ou non (témoin) le composé à l'essai ou la référence (CaCl₂ à 1,5 mM) et les cellules ont été incubées pendant 48 heures. Toutes les conditions expérimentales ont été réalisées en n=3, pour chaque marqueur.

Après incubation, le milieu d'essai a été éliminé et les cellules ont été rincées et congelées à -80 °C.

L'expression des marqueurs a été évaluée par RT-qPCR sur les ARN messagers (ARNm) extraits des tapis cellulaires de chaque traitement (les réplicats ont été poolés avant l'extraction de l'ARN). Les étapes d'extraction d'ARN, réverse transcription, amorces et conditions de PCR ont été décrites précédemment¹⁻³.

### 2) Résultats

### Screening

Les résultats sont présentés dans le tableau 10 ci-dessous qui illustre le niveau d'expression de la TGK via le marquage fluorescent des NHEK, l'intensité de fluorescence des NHEK étant proportionnelle à l'expression de TGK.

**Tableau 10**

| Composé testé | Témoin | EX1MF1046 0,005mg/ml | EX2MF1046 0,003mg/ml | EX3MF1046 0,005mg/ml | EX4MF1046 0,005mg/ml | EX13MF1046 0,001mg/ml |
|---|---|---|---|---|---|---|
| Taux d'expression de TGK | / | ++ | ++ | ++++ | ++ | / |

L'extrait EX3MF1046 se révèle le plus actif dans ces conditions.

### Influence de l'extrait EX3MF1046 à différentes concentrations, sur l'expression de TGK

Le tableau 11 illustre le niveau d'expression de la TGK via le marquage fluorescent des NHEK pour différentes concentration de l'extrait EX3MF1046.

**Tableau 11**

| Traitement | | Expression TGK^{a)} | | Témoin | |
|---|---|---|---|---|---|
| Composé testé | Concentration | Moyenne | ESM | % | ESM |
| Témoin | - | 22042 | 3399 | 100 | 15 |
| CaCl₂ | 1,5mM | 69610 | 681 | 316 | 3 |
| EX3MF1046 | 0,0016mg/ml | 41326 | 3696 | 187 | 17 |
| | 0,005mg/ml | 133494 | 4753 | 606 | 22 |
| | 0,01mg/ml | 201936 | 7097 | 916 | 32 |
| | 0,02mg/ml | 320616 | 6643 | 1455 | 30 |

| | | | | | |
|---|---|---|---|---|---|
| L'expression de TGK est donnée en Intensité de fluorescence/Nombre de cellules (UA) | | | | | |

Ces résultats montrent que l'extrait EX3MF1046 induit de façon dose-dépendante l'expression de TGK dans les cultures de NHEK. L'activité est supérieure à celle de la référence chlorure de calcium, dès la concentration de 5 µg/ml ; l'effet maximum qui est 4 fois supérieur à celui du CaCl₂ est observé à la concentration non cytotoxique de 20 µg/ml.

### Influence de l'extrait EX10MF1046, sur l'expression de la TKG, la filaggrine et la KRT10

La même méthodologie que celle employée pour évaluer l'effet d'un extrait sur l'expression de TGK a été appliquée pour les marqueurs filaggrine et KRT10.

Le tableau 12 présente une évaluation du niveau d'expression des trois marqueurs ci-dessus via le marquage fluorescent des NHEK.

**Tableau 12**

| Traitement | | Expression TKG | Expression filaggrine | Expression KRT10 |
|---|---|---|---|---|
| Composé testé | Concentration | | | |
| Témoin | - | +/- | - | + |
| CaCl₂ | 1,5mM | +++ | +++ | +++ |
| EX10MF1046 | 20µg/ml | ++++ | ++ | ++++ |

On observe que la filtration de l'extrait EX3MF1046 maintient l'effet pro-différenciant de l'extrait.

Dans cette expérience, on retrouve l'effet inducteur de TGK et on observe une très forte surexpression de protéine KRT10. Contrairement au calcium qui focalise l'effet sur quelques cellules en les transformant en très grosses cellules différenciées (cornéocytes), EX10MF1046 induit une très forte surexpression de KRT10 dans de nombreuses cellules mais n'augmente pas significativement la taille des cellules. Le mécanisme d'action du calcium et de EX10MF1046 sont donc différents et pourraient être additionnels ou synergiques. Ceci est confirmé par l'analyse de la filaggrine, qui augmente (plus modérément) suite au traitement par EX10MF1046, mais sans induire la production caractéristique de granules de filaggrine dans les cellules répondant au calcium.

### Influence de l'extrait EX10MF1046, sur l'expression d'autres marqueurs de la différenciation épidermique

L'analyse a été élargie à d'autres marqueurs protéiques de la différenciation épidermique, à savoir, la loricine, l'involucrine et la kératine 1 (KRT1).

L'influence de EX10MF1046 à 20 µg/ml est étudiée sur l'expression des transcrits des différents marqueurs ci-dessus par les cultures de kératinocytes à l'état basal. Les traitements sont de 48 h, l'analyse est réalisée par RT-qPCR. Les résultats sont donnés en % des témoins non traités après randomisation des expressions relatives par rapport au gène de ménage GAPDH. Ils sont représentés sur la figure 5.

Les analyses en RT-qPCR montrent une surexpression très nette de tous les marqueurs de différenciation en présence de l'extrait; les stimulations étaient fortes (stimulation d'un facteur 4 à plusieurs dizaines de fois selon les marqueurs) et toutes supérieurs à celles obtenues avec le calcium, à l'exception du marqueur involucrine.

### Exemple 6 : Evaluation d'extraits de salicaire dans des épidermes humains reconstruits (RHE)

### 1) Matériels et méthodes

### Epidermes humains reconstruits

RHE produits à partir de kératinocytes de prépuce humain et largement décrits dans les publications de BIO*alternatives*¹⁻⁴.

Les RHE sont cultivés à 37°C, 5% CO₂, à l'interface air-liquide, en milieu Epilife + calcium 1,5 mM et compléments, sans vitamine C. La référence pour les études d'effets pro-différenciants est la vitamine C (50 µg/ml).

### Extraits testés

L'extrait EX10MF1046 de l'exemple 1 a été testé à 20 µg/ml, dans le milieu de culture des RHE.

### Immunomarquages in situ

Les RHE sont placés à l'interface air-liquide (J0) et cultivés pendant 7 jours en présence ou absence (témoin) de l'extrait EX10MF1046 ou de la vitamine C (référence).

A la fin de l'incubation, les épidermes sont rincés puis fixés dans une solution de formaldéhyde. Les tissus fixés sont déshydratés par des bains d'éthanol successifs et croissants puis inclus en paraffine « Paraplast ». Des coupes transversales sont réalisées au microtome (épaisseur 5 µm) puis maintenues à température ambiante jusqu'à la réalisation des marquages.

Les coupes sont déparaffinées puis les sites antigéniques sont démasqués dans une solution de tampon citrate pH6 (DAKO, S1700). Après lavage en PBS-T, les coupes sont incubées avec une solution de peroxyde d'hydrogène 3% durant 5 minutes. Après lavage, les coupes sont ensuite incubées à température ambiante pendant 1 heure avec l'anticorps primaire (anti-TGK, SC-25786 ; anti-KRT10, SC-23877 ; anti-filaggrine, SC-66192 ; tous de chez Santa-Cruz Biotech). Après lavage, le marquage est révélé avec un kit de détection streptavidine-peroxydase (DAKO, réf. K0690) couplé au chromogène AEC (DAKO, K346111). Après contre-coloration à l'hématoxyline et lavage en eau ultrapure, les coupes sont montées entre lame et lamelle en milieu aqueux Glycergel (DAKO, C056330). Les coupes sont observées à l'aide d'un microscope NIKON E400. Les images numériques sont enregistrées avec une caméra NIKON DS-Ri1 et le logiciel NIS-Elements 3.10.

### 2) Résultats

Le passage à l'interface air-liquide conduit, dans les conditions optimales de reconstruction épidermique, à une très forte différenciation qu'il est très difficile de sur-stimuler. Aussi il convient de se mettre en conditions non-optimales ou légèrement carencées pour voir l'effet de produits stimulant la différenciation.

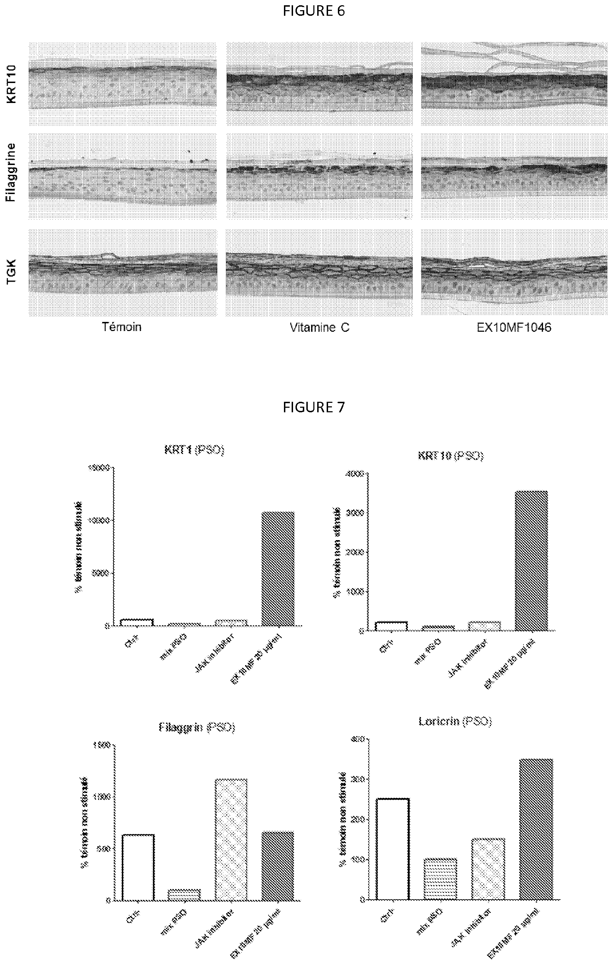

Les images numériques obtenues sont présentées à la figure 6.

Dans ces conditions expérimentales présentant une carence (vitamine C), l'extrait EX10MF1046 montre une nette compensation et une forte stimulation de l'expression de KRT10, filaggrine et TGK.

### Exemple 7 : Effets protecteur d'extraits de salicaire sur des lésions cutanées induites par l'inflammation (psoriasis, dermatites) de l'épiderme ; évaluation dans des épidermes reconstruits (RHE)

### 1) Matériels et méthodes

### Cellules et épidermes reconstruits

Les NHEK sont préparés comme dans l'exemple 5.

Les RHE sont préparés comme dans l'exemple 6 (milieu complet incluant vitamine C).

### Mélange inflammatoire, référence

Le mélange inflammatoire (mix cytokines, M3) est constitué d'interleukine-17 (IL-17), oncostatine M (OSM) et tumor-necrosis factor alpha (TNFα), toutes provenant de chez R&D Systems, à la concentration finale de 3 ng/ml chaque.

La référence est le « JAK inhibitor 1 » (Calbiochem CAS 457081-03-7) à 10 µM final.

### Extrait testé

L'extrait EX10MF1046 de l'exemple 1 est testé à 20 µg/ml, dans le milieu de culture des RHE.

### Analyse des transcrit, RT-qPCR

Les NHEK sont pré-cultivés comme dans l'exemple 5 puis traités pendant 24 h avec l'extrait EX10MF1046 (20 µg/ml) ou le JAK inhibitor (10 µM). Le mélange de cytokines est ensuite ajouté aux milieux contenant ou non l'extrait et les cultures sont poursuivies pendant 24 h.

A la fin de l'incubation, les tapis cellulaires sont rincés puis extraits en tampon de lyse ; les protocoles et analyses suivants sont les mêmes que ceux de l'exemple 5.

Les résultats sont donnés en % des témoins non traités, ils sont représentés à la figure 7.

### Immunomarquages in situ

Les RHE sont placés à l'interface air-liquide (J0) et cultivés jusqu'à J11. Puis les RHE sont traités en systémique avec l'extrait EX10MF1046 (20 µg/ml) ou le JAK inhibitor (10 µM) pendant 7h. Le mélange de cytokines est ajouté aux milieux contenant ou non les produits et les cultures sont poursuivies pendant 48 h.

Le traitement des tissus, les marquages et l'analyse sont réalisés comme dans l'exemple 6. Des images numériques des coupes observées sont enregistrées.

Les NHEK ou les RHE sont traités par l'extrait EX10MF1046 ou la référence, puis par un mélange cytokinique constitué par au moins une cytokine activant la voie Jak/Stat, soit OSM ou IL-22 (activateurs de STAT-3/1) ; un activateur de voie NF-kB, soit IL-1 ou TNF-α et une troisième cytokine pouvant être l'IL-17 (activateur de CREBP) orientant vers une réponse de type psoriasis ou l'IL-4/IL-13 (activateur de STAT-6), orientant vers la dermatite atopique ⁵⁻⁷. A noter que toutes ces cytokines sont aussi plus ou moins activatrices des voies MAKPkinases (mitogen-activated protein kinases) chez le kératinocyte. Dans ces mélanges, les cytokines inductrices de STAT-3/1 (OSM, IL-22) ^{2-3,7} sont cruciales dans la physiopathologie cutanée, elles sont responsables de l'effet sur la différenciation cutanée et des modifications morphologiques observées dans ces pathologies. Les autres cytokines, notamment IL-17, TNF-a et IL-1 ⁵⁻⁷ sont plus impliquées et de façon hautement synergique dans l'induction de l'immunité innée (peptides antimicrobiens) et la production de chimiokines responsables du maintien et de l'amplification de la boucle inflammatoire responsable de ces pathologies chroniques ⁵⁻⁷.

Ce mélange choisi contient l'OSM comme inducteur de STAT-3/1, associé au TNF-a (NF-kB, MAPkinases) et à l'IL-17 (pour donner l'orientation « psoriasis », Th17. Ce mélange a un effet inflammatoire hautement synergique qui mime donc la pathologie inflammatoire cutanée au niveau de l'épiderme et en particulier le psoriasis. Le remplacement de L'IL-17 (cytokine Th17) par une ou plusieurs cytokines Th2 (IL-4/IL-13) conduirait à un phénotype cutané plus proche de la dermatite atopique⁶. Dans les deux cas, la différenciation anormale de l'épiderme est remarquable, avec perte des marqueurs de différenciation terminale, hyperplasie (etc).

La référence est le « JAK inhibitor 1 », inhibiteur large de voie Jak-Stat et testé à forte concentration.

### Dosages ELISA

Les milieux de culture des RHE à 48h sont prélevés et congelés à -80°C. Le contenu en IL-8 a été dosé à l'aide d'un kit ELISA spécifique (R&D Systems DY208), selon le protocole préconisé par le fournisseur.

### 2) Résultats

L'influence de l'extrait EX10MF1046 à 20 µg/ml est étudiée sur l'expression des transcrits des marqueurs KRT1, KRT10, filaggrine et loricrine, par les cultures de kératinocytes stimulées (ou non (Ctrl-) par un mélange de cytokines (IL-17, OSM, TNFα à 3 ng/ml, mix PSO). Les traitements sont de 48 h au total, l'analyse est réalisée par RT-qPCR.

Les résultats sont représentés sur la figure 7.

Ils montrent que l'extrait EX10MF1046 protège l'épiderme des effets de l'inflammation en restaurant l'expression des marqueurs de différenciation tardive, filaggrine et loricrine, et que cet extrait induit une forte surexpression des kératines KRT1 et KRT10 dans ces conditions. L'extrait EX10MF1046 protège donc potentiellement des défauts de différenciation de l'épiderme induits par l'inflammation.

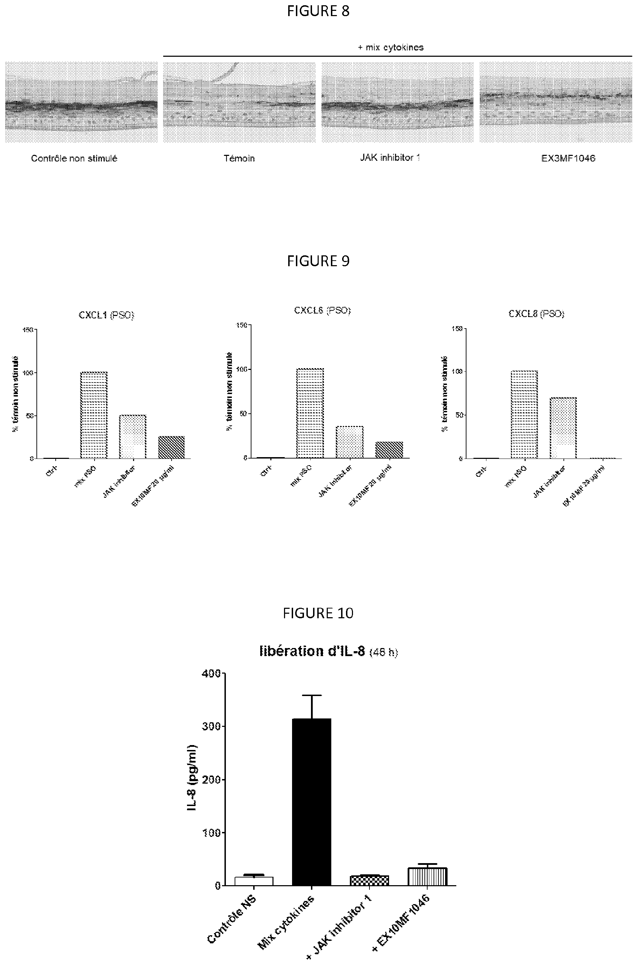

L'expression de KRT10 par des épidermes reconstruits (RHE analyse à J13) stimulés par le mélange de cytokines IL-17, OSM, TNFα est observée sur les images numériques représentées à la figure 8.

Cette figure 8 montre une restauration partielle mais nette de l'expression de la protéine KRT10 par le traitement avec l'extrait EX10MF1046 dans les RHE traités par le mélange pro-inflammatoire.

La figure 9 montre l'effet inhibiteur de l'extrait EX10MF1046 (à 20µg/ml) de l'expression de chimiokines induite par l'inflammation des kératinocytes. Cet effet résulte de l'interruption de la boucle inflammatoire en empêchant le recrutement de leucocytes au niveau de la peau lésionnelle. Cet effet s'ajoute aux effets sur la différenciation (figure 7) pour contribuer au retour à une homéostasie normale de la peau (résolution de l'inflammation).

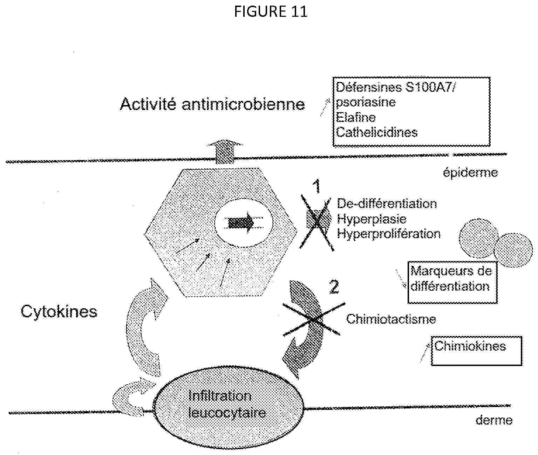

D'autre part, comme cela a été vu au niveau des transcrits (figure 9), EX10MF1046 inhibe très fortement l'expression/libération de chimiokines et notamment l'IL-8 au niveau de la protéine (figure 10), confirmant son effet inhibiteur potentiel du recrutement de leucocytes (polynucléaires dans le cas de l'IL-8) au niveau des lésions cutanées (en plus de l'effet positif sur ces mêmes lésions). Ces effets 1) protecteurs des lésions cutanées et 2) limitant l'infiltration leucocytaire dans la peau sont résumés dans la figure 11 qui représente schématiquement une boucle d'inflammation chronique de la peau (psoriasis...).

EX10MF1046 protège donc des effets délétères (lésions cutanées) des cytokines produites par les leucocytes (1), tout en bloquant le recrutement des infiltrats leucocytaires et cassant ainsi le cercle vicieux de l'inflammation chronique (2).

### Exemple 8 : Effets d'extraits de salicaire sur le renforcement du cheveu

### 1) Matériels et méthodes

### Cultures de follicules de cheveu humain in vitro

Les bulbes de cheveux sont microdisséqués à partir d'un lifting et placés dès l'isolement en milieu de culture (Milieu E de William complémenté ; Invitrogen) contenant ou non (témoin), l'extrait à l'essai puis incubés pendant 72 heures. Pour chaque condition, un excès de bulbes est préparé afin d'atteindre la valeur cible de 6 cheveux/condition sélectionnés en final.

En fin d'incubation, les cheveux sont congelés à sec en azote liquide et stockés à - 80°C jusqu'à l'extraction d'ARN.

### Extrait testé

L'extrait EX10MF1046 de l'exemple 1 est testé à 20 µg/ml, dans le milieu de culture des RHE.

### Analyse des transcrit, RT-qPCR

L'isolement et le contrôle qualité des ARN sont réalisés comme dans l'exemple 5.

L'amplification des ARN et la synthèse des analogues d'ARN biotinylés (ARNa) sont réalisés à l'aide du kit « GeneChip 3'IVT Express » (Affymetrix®). L'hybridation et le marquage sont réalisés à l'aide du kit « GeneAtlas™ hybridization, wash and stain kit for 3'IVT arrays » (Affymetrix®). L'hybridation des ARNa fragmentés sur la puce Affymetrix® HG-U219 est réalisée sur la station d'hybridation « GeneAtlas™ fluidics station » (Affymetrix®) pendant 20 heures à 45°C. Toutes les procédures expérimentales et de normalisation sont réalisées selon les directives du fournisseur.

L'analyse est effectuée par microarray full transcriptome sur puce Affymetrix® HG-U219.

### 2) Résultats

Les kératines sont des composants majeurs de la tige pilaire. Une augmentation de ces kératines aura potentiellement un impact positif sur le développement/renforcement capillaire.

L'effet de l'extrait EX10MF1046 par rapport au témoin est illustré dans le tableau 13 suivant qui donne l'expression de transcrits de marqueurs de différenciation dans des follicules de cheveu humain maintenus en survie *in vitro.*

**Tableau 13**

| **Affy U219** | **Témoin** | **Salicaire** | | **Nom du gène** | **Abréviation** |
|---|---|---|---|---|---|
| Probe Set ID | **RE1** | **RE2** | **Facteur de modulation** | | |
| 11723952_at | 120.1177 | 441.5676 | 3.68 | keratin 1 | KRT1 |
| 11757401_a_at | 1557.519 | 3385.916 | 2.17 | keratin 10 | KRT10 |
| 11731852_at | 54.47806 | 114.4569 | 2.10 | loricrin | LOR |
| 11738505_at | 1956.021 | 3712.088 | 1.90 | keratin associated protein 13-1 | KRTAP13-1 |
| 11757905_x_at | 295.5005 | 513.2589 | 1.74 | keratin 18 | KRT18 |
| 11759172_at | 40.7767 | 70.12949 | 1.72 | keratin associated protein 19-4 | KRTAP19-4 |
| 11759392_at | 59.00863 | 100.2564 | 1.70 | keratin associated protein 21-2 | KRTAP21-2 |
| 11731500_a_at | 264.1598 | 431.7103 | 1.63 | keratin 19 | KRT19 |
| 11738284_at | 2832.61 | 4332.157 | 1.53 | keratin associated protein 13-2 | KRTAP13-2 |

Cette expérience montre que les transcrits de certaines kératines et protéines associées ont une tendance nette à l'augmentation d'expression, comme la KRT1, la KRT10 et la loricrine. On retrouve en cela les effets sur les kératinocytes. Les effets sont moins visibles que dans les exemples précédents du fait 1) que le follicule est moins sensible aux traitements car « protégé » par les feuillets internes et externes (les kératinocytes ne sont pas en contact direct avec le milieu) et par son propre volume (accessibilité aux kératinocytes internes au follicule) et 2) que la méthode utilisée par microarrays est moins sensible que la qPCR.

### BIBLIOGRAPHIQUES

1) Mcheik JN, Barrault C, Pedretti N, Garnier J, Juchaux F, Levard G, Morel F, Lecron JC, Bernard FX. Foreskin-isolated keratinocytes provide successful extemporaneous autologous paediatric skin grafts. J Tissue Eng Regen Med. 2013 Mar 14. doi: 10.1002/term.1690. [Epub ahead of print]
2) Boniface K, Bernard FX, Garcia M, Gurney AL, Lecron JC, Morel F. IL-22 inhibits epidermal differentiation and induces proinflammatory gene expression and migration of human keratinocytes. J Immunol. 2005 Mar 15;174(6):3695-702.
3) Boniface K, Diveu C, Morel F, Pedretti N, Froger J, Ravon E, Garcia M, Venereau E, Preisser L, Guignouard E, Guillet G, Dagregorio G, Pène J, Moles JP, Yssel H, Chevalier S, Bernard FX, Gascan H, Lecron JC. Oncostatin M secreted by skin infiltrating T lymphocytes is a potent keratinocyte activator involved in skin inflammation. J Immunol. 2007 Apr 1;178(7):4615-22.
4) Guenou H, Nissan X, Larcher F, Feteira J, Lemaitre G, Saidani M, Del Rio M, Barrault CC, Bernard FX, Peschanski M, Baldeschi C, Waksman G. Human embryonic stem-cell derivatives for full reconstruction of the pluristratified epidermis: a preclinical study. Lancet. 2009 Nov 21;374(9703):1745-53
5) Guilloteau K, Paris I, Pedretti N, Boniface K, Juchaux F, Huguier V, Guillet G, Bernard FX, Lecron JC, Morel F.Skin Inflammation Induced by the Synergistic Action of IL-17A, IL-22, Oncostatin M, IL-1 alpha, and TNF-alpha Recapitulates Some Features of Psoriasis. J Immunol. 2010 Mar 24. [Epub ahead of print]
6) Bernard FX, Morel F, Camus M, Pedretti N, Barrault C, Garnier J, Lecron JC. Keratinocytes under Fire of Proinflammatory Cytokines: Bona Fide Innate Immune Cells Involved in the Physiopathology of Chronic Atopic Dermatitis and Psoriasis. J Allergy (Cairo). 2012;2012:718725. doi: 10.1155/2012/718725. Epub 2012 Nov 5.
7) US2012142755A1 différenciation cutanée et des modifications morphologiques observées dans ces pathologies. Les autres cytokines, notamment IL-17, TNF-a et IL-1 ⁵⁻⁷ sont plus impliquées et de façon hautement synergique dans l'induction de l'immunité innée (peptides antimicrobiens) et la production de chimiokines responsables du maintien et de l'amplification de la boucle inflammatoire responsable de ces pathologies chroniques ⁵⁻⁷.

Ce mélange choisi contient l'OSM comme inducteur de STAT-3/1, associé au TNF-a (NF-kB, MAPkinases) et à l'IL-17 (pour donner l'orientation « psoriasis », Th17. Ce mélange a un effet inflammatoire hautement synergique qui mime donc la pathologie inflammatoire cutanée au niveau de l'épiderme et en particulier le psoriasis. Le remplacement de L'IL-17 (cytokine Th17) par une ou plusieurs cytokines Th2 (IL-4/IL-13) conduirait à un phénotype cutané plus proche de la dermatite atopique⁶. Dans les deux cas, la différenciation anormale de l'épiderme est remarquable, avec perte des marqueurs de différenciation terminale, hyperplasie (etc).

La référence est le « JAK inhibitor 1 », inhibiteur large de voie Jak-Stat et testé à forte concentration.

### Dosages ELISA

Les milieux de culture des RHE à 48h sont prélevés et congelés à -80°C. Le contenu en IL-8 a été dosé à l'aide d'un kit ELISA spécifique (R&D Systems DY208), selon le protocole préconisé par le fournisseur.

### 2) Résultats

L'influence de l'extrait EX10MF1046 à 20 µg/ml est étudiée sur l'expression des transcrits des marqueurs KRT1, KRT10, filaggrine et loricrine, par les cultures de kératinocytes stimulées (ou non (Ctrl-) par un mélange de cytokines (IL-17, OSM, TNFα à 3 ng/ml, mix PSO). Les traitements sont de 48 h au total, l'analyse est réalisée par RT-qPCR.

Les résultats sont représentés sur la figure 7.

Ils montrent que l'extrait EX10MF1046 protège l'épiderme des effets de l'inflammation en restaurant l'expression des marqueurs de différenciation tardive, filaggrine et loricrine, et que cet extrait induit une forte surexpression des kératines KRT1 et KRT10 dans ces conditions. L'extrait EX10MF1046 protège donc potentiellement des défauts de différenciation de l'épiderme induits par l'inflammation.

L'expression de KRT10 par des épidermes reconstruits (RHE analyse à J13) stimulés par le mélange de cytokines IL-17, OSM, TNFα est observée sur les images numériques représentées à la figure 8.

Cette figure 8 montre une restauration partielle mais nette de l'expression de la protéine KRT10 par le traitement avec l'extrait EX10MF1046 dans les RHE traités par le mélange pro-inflammatoire.

La figure 9 montre l'effet inhibiteur de l'extrait EX10MF1046 (à 20µg/ml) de l'expression de chimiokines induite par l'inflammation des kératinocytes. Cet effet résulte de l'interruption de la boucle inflammatoire en empêchant le recrutement de leucocytes au niveau de la peau lésionnelle. Cet effet s'ajoute aux effets sur la différenciation (figure 7) pour contribuer au retour à une homéostasie normale de la peau (résolution de l'inflammation).

D'autre part, comme cela a été vu au niveau des transcrits (figure 9), EX10MF1046 inhibe très fortement l'expression/libération de chimiokines et notamment l'IL-8 au niveau de la protéine (figure 10), confirmant son effet inhibiteur potentiel du recrutement de leucocytes (polynucléaires dans le cas de l'IL-8) au niveau des lésions cutanées (en plus de l'effet positif sur ces mêmes lésions). Ces effets 1) protecteurs des lésions cutanées et 2) limitant l'infiltration leucocytaire dans la peau sont résumés dans la figure 11 qui représente schématiquement une boucle d'inflammation chronique de la peau (psoriasis...).

EX10MF1046 protège donc des effets délétères (lésions cutanées) des cytokines produites par les leucocytes (1), tout en bloquant le recrutement des infiltrats leucocytaires et cassant ainsi le cercle vicieux de l'inflammation chronique (2).

### Exemple 8 : Effets d'extraits de salicaire sur le renforcement du cheveu

### 1) Matériels et méthodes

### Cultures de follicules de cheveu humain in vitro

Les bulbes de cheveux sont microdisséqués à partir d'un lifting et placés dès l'isolement en milieu de culture (Milieu E de William complémenté; Invitrogen) contenant ou non (témoin), l'extrait à l'essai puis incubés pendant 72 heures. Pour chaque condition, un excès de bulbes est préparé afin d'atteindre la valeur cible de 6 cheveux/condition sélectionnés en final.

En fin d'incubation, les cheveux sont congelés à sec en azote liquide et stockés à - 80°C jusqu'à l'extraction d'ARN.

### Extrait testé

L'extrait EX10MF1046 de l'exemple 1 est testé à 20 µg/ml, dans le milieu de culture des RHE.

### Analyse des transcrit, RT-qPCR

L'isolement et le contrôle qualité des ARN sont réalisés comme dans l'exemple 5.

L'amplification des ARN et la synthèse des analogues d'ARN biotinylés (ARNa) sont réalisés à l'aide du kit « GeneChip 3'IVT Express » (Affymetrix®). L'hybridation et le marquage sont réalisés à l'aide du kit « GeneAtlas™ hybridization, wash and stain kit for 3'IVT arrays » (Affymetrix®). L'hybridation des ARNa fragmentés sur la puce Affymetrix® HG-U219 est réalisée sur la station d'hybridation « GeneAtlas™ fluidics station » (Affymetrix®) pendant 20 heures à 45°C. Toutes les procédures expérimentales et de normalisation sont réalisées selon les directives du fournisseur.

L'analyse est effectuée par microarray full transcriptome sur puce Affymetrix® HG-U219.

### 2) Résultats

Les kératines sont des composants majeurs de la tige pilaire. Une augmentation de ces kératines aura potentiellement un impact positif sur le développement/renforcement capillaire.

L'effet de l'extrait EX10MF1046 par rapport au témoin est illustré dans le tableau 13 suivant qui donne l'expression de transcrits de marqueurs de différenciation dans des follicules de cheveu humain maintenus en survie *in vitro.*

**Tableau 13**

| **Affy U219** | **Témoin** | **Salicaire** | | **Nom du gène** | **Abréviation** |
|---|---|---|---|---|---|
| Probe Set ID | **RE1** | **RE2** | **Facteur de modulation** | | |
| 11723952_at | 120.1177 | 441.5676 | 3.68 | keratin 1 | KRT1 |
| 11757401_a_at | 1557.519 | 3385.916 | 2.17 | keratin 10 | KRT10 |
| 11731852_at | 54.47806 | 114.4569 | 2.10 | loricrin | LOR |
| 11738505_at | 1956.021 | 3712.088 | 1.90 | keratin associated protein 13-1 | KRTAP13-1 |
| 11757905_x_at | 295.5005 | 513.2589 | 1.74 | keratin 18 | KRT18 |
| 11759172_at | 40.7767 | 70.12949 | 1.72 | keratin associated protein 19-4 | KRTAP19-4 |
| 11759392_at | 59.00863 | 100.2564 | 1.70 | keratin associated protein 21-2 | KRTAP21-2 |
| 11731500_a_at | 264.1598 | 431.7103 | 1.63 | keratin 19 | KRT19 |
| 11738284_at | 2832.61 | 4332.157 | 1.53 | keratin associated protein 13-2 | KRTAP13-2 |

Cette expérience montre que les transcrits de certaines kératines et protéines associées ont une tendance nette à l'augmentation d'expression, comme la KRT1, la KRT10 et la loricrine. On retrouve en cela les effets sur les kératinocytes. Les effets sont moins visibles que dans les exemples précédents du fait 1) que le follicule est moins sensible aux traitements car « protégé » par les feuillets internes et externes (les kératinocytes ne sont pas en contact direct avec le milieu) et par son propre volume (accessibilité aux kératinocytes internes au follicule) et 2) que la méthode utilisée par microarrays est moins sensible que la qPCR.

### BIBLIOGRAPHIQUES

1) Mcheik JN, Barrault C, Pedretti N, Garnier J, Juchaux F, Levard G, Morel F, Lecron JC, Bernard FX. Foreskin-isolated keratinocytes provide successful extemporaneous autologous paediatric skin grafts. J Tissue Eng Regen Med. 2013 Mar 14. doi: 10.1002/term.1690. [Epub ahead of print]
2) Boniface K, Bernard FX, Garcia M, Gurney AL, Lecron JC, Morel F. IL-22 inhibits epidermal differentiation and induces proinflammatory gene expression and migration of human keratinocytes. J Immunol. 2005 Mar 15;174(6):3695-702.
3) Boniface K, Diveu C, Morel F, Pedretti N, Froger J, Ravon E, Garcia M, Venereau E, Preisser L, Guignouard E, Guillet G, Dagregorio G, Pène J, Moles JP, Yssel H, Chevalier S, Bernard FX, Gascan H, Lecron JC. Oncostatin M secreted by skin infiltrating T lymphocytes is a potent keratinocyte activator involved in skin inflammation. J Immunol. 2007 Apr 1;178(7):4615-22.
4) Guenou H, Nissan X, Larcher F, Feteira J, Lemaitre G, Saidani M, Del Rio M, Barrault CC, Bernard FX, Peschanski M, Baldeschi C, Waksman G. Human embryonic stem-cell derivatives for full reconstruction of the pluristratified epidermis: a preclinical study. Lancet. 2009 Nov 21;374(9703):1745-53
5) Guilloteau K, Paris I, Pedretti N, Boniface K, Juchaux F, Huguier V, Guillet G, Bernard FX, Lecron JC, Morel F.Skin Inflammation Induced by the Synergistic Action of IL-17A, IL-22, Oncostatin M, IL-1 alpha, and TNF-alpha Recapitulates Some Features of Psoriasis. J Immunol. 2010 Mar 24. [Epub ahead of print]
6) Bernard FX, Morel F, Camus M, Pedretti N, Barrault C, Garnier J, Lecron JC. Keratinocytes under Fire of Proinflammatory Cytokines: Bona Fide Innate Immune Cells Involved in the Physiopathology of Chronic Atopic Dermatitis and Psoriasis. J Allergy (Cairo). 2012;2012:718725. doi: 10.1155/2012/718725. Epub 2012 Nov 5.
7) US2012142755A1

## Revendications

1. Utilisation cosmétique non thérapeutique de la vescalagine, de la castalagine, ou d'un extrait de Lythrum salicaria L. contenant de l'acide gallique, de la vescalagine et de la castalagine, pour stimuler la fonction barrière de l'épiderme.

2. Utilisation cosmétique non thérapeutique selon la revendication 1, pour apaiser les peaux sèches à tendance atopique et/ou pour améliorer et/ou renforcer l'hydratation de la peau.

3. Utilisation de la vescalagine, de la castalagine, ou d'un extrait de Lythrum salicaria L. contenant de l'acide gallique, de la vescalagine et de la castalagine, comme ingrédient actif dans une composition cosmétique destinée à être appliquée sur une peau sèche à tendance atopique, à la lutte contre le vieillissement de la peau, à la lutte contre les démangeaisons et contre le prurit, à l'hydratation et à la protection contre les agressions externes liées à la pollution et au stress.

4. Utilisation de la vescalagine, de la castalagine, ou d'un extrait de Lythrum salicaria L. contenant de l'acide gallique, de la vescalagine et de la castalagine, comme ingrédient actif dans une composition cosmétique destinée à être appliquée sur les cheveux, en tant qu'agent protecteur ou réparateur.

5. Utilisation selon la revendication 3 ou 4, **caractérisée en ce que** la composition cosmétique contient de 0,001 à 5% en poids de vescalagine, de castalagine ou dudit extrait, par rapport au poids total de la composition.

6. Composé ou sel de ce composé, ledit composé étant choisi parmi la vescalagine, de la castalagine, ou extrait de Lythrum salicaria L. contenant de l'acide gallique, de la vescalagine et de la castalagine, pour utilisation dans le traitement des lésions provoquées dans la paroi intestinale, par la maladie de Crohn.

## Patentansprüche

1. Nichttherapeutische, kosmetische Verwendung von Vescalagin, von Castalagin oder von einem Extrakt aus Lythrum salicaria L., der Gallussäure, Vescalagin und Castalagin enthält, zum Anregen der Barrierefunktion der Haut.

2. Nichttherapeutische, kosmetische Verwendung nach Anspruch 1 zum Beruhigen von trockener Haut mit atopischer Tendenz und/oder zum Verbessern und/oder Stärken der Feuchtigkeitsversorgung der Haut.

3. Verwendung von Vescalagin, von Castalagin oder von einem Extrakt aus Lythrum salicaria L., der Gallussäure, Vescalagin und Castalagin enthält, als Wirkstoff in einer kosmetischen Zusammensetzung, die zum Auftragen auf einer trockenen Haut mit atopischer Tendenz, zum Kampf gegen Hautalterung, zum Kampf gegen Juckreiz und gegen Pruritus, zur Feuchtigkeitsversorgung und zum Schutz gegen äußere Einflüsse in Verbindung mit Verschmutzung und Stress bestimmt ist.

4. Verwendung von Vescalagin, von Castalagin oder von einem Extrakt aus Lythrum salicaria L., der Gallussäure, Vescalagin und Castalagin enthält, als Wirkstoff in einer kosmetischen Zusammensetzung, die zum Auftragen auf den Haaren als Schutz- oder Reparaturmittel bestimmt ist.

5. Verwendung nach Anspruch 3 oder 4, **dadurch gekennzeichnet, dass** die kosmetische Zusammensetzung von 0,001 bis 5 Gew.-% an Vescalagin, an Castalagin oder von dem Extrakt in Bezug auf das Gesamtgewicht der Zusammensetzung enthält.

6. Verbindung oder Salz aus dieser Verbindung, wobei die Verbindung gewählt wird aus Vescalagin, aus Castalagin oder einem Extrakt aus Lythrum salicaria L., der Gallussäure, Vescalagin und Castalagin enthält, zur Verwendung in der Behandlung von Verletzungen, die durch Morbus Crohn in der Darmwand verursacht werden.

## Claims

1. A non-therapeutic cosmetic use of vescalagin, castalagin, or an extract of Lythrum salicaria L. containing gallic acid, vescalagin and castalagin, to stimulate the barrier function of the epidermis.

2. The non-therapeutic cosmetic use according to claim 1, for soothing dry skins with an atopic tendency and/or for improving and/or reinforcing the hydration of the skin.

3. A use of vescalagin, castalagin, or an extract of Lythrum salicaria L. containing gallic acid, vescalagin and castalagin, as active ingredient in a cosmetic composition intended to be applied to a dry skin with an atopic tendency, to control skin ageing, to control itching and pruritus, to hydration and to protection against external aggressions related to pollution and stress.

4. A use of vescalagin, castalagin, or an extract of Lythrum salicaria L. containing gallic acid, vescalagin and castalagin, as active ingredient in a cosmetic composition intended to be applied to the hair, as a protective or repairing agent.

5. The use according to claim 3 or 4, **characterized in that** the cosmetic composition contains from 0.001 to 5 weight% of vescalagin, castalagin or of said extract, with respect to the total weight of the composition.

6. A Compound or salt of this compound, said compound being selected from vescalagin, castalagin, or extract of Lythrum salicaria L. containing gallic acid, vescalagin and castalagin, for use in the treatment of lesions caused in the intestinal wall, by Crohn's disease.
